# EUROPEAN PATENT APPLICATION

(11) **EP 4 296 921 A1**
(43) Date of publication of application: **27.12.2023**
(21) Application number: 22180160.8
(22) Date of filing: 21.06.2022
(51) Int. Cl.: G06Q 10/08, G16H 20/60

(54) **MILK MANAGEMENT METHOD, INFANT MILK CONTAINER ATTACHMENT AND SYSTEM**

(71) Applicant: Koninklijke Philips N.V., 5656 AG Eindhoven (NL)
(72) Inventor: BOURQUIN, Yannyk Parulian Julian, Eindhoven (NL); VAN BENTUM, Jesper William, Eindhoven (NL); LONG, Xi, Eindhoven (NL); BROCKHUIS, Lili-Marjan, Eindhoven (NL); WIJNOLTZ, Anna Louise, Eindhoven (NL)
(74) Representative: Philips Intellectual Property & Standards

(57) **Abstract**

Provided is a computer implemented method (100) for communicating an infant milk management recommendation to a user. The method comprises monitoring (102), overtime, data indicative of a temperature of each quantity of milk in a reserve of infant milk. The infant milk management recommendation is determined (104) at least partly based on said temperature sensor data, and the recommendation is communicated (106) to the user. Further provided is a computer program for implementing the method, an infant milk container attachment, and an infant milk management system.

## Description

### FIELD OF THE INVENTION

This invention relates to a method for communicating an infant milk management recommendation to a user, and a computer program for implementing the method. The invention further relates to an infant milk container attachment, and an infant milk management system.

### BACKGROUND OF THE INVENTION

Over the past decades, evidence for the health advantages of breastfeeding and recommendations for practice have continued to grow. The World Health Organization (WHO) considers breastfeeding to reduce child mortality and to have health benefits that extend into adulthood. On a population basis, exclusive breastfeeding for the first six months of life is recommended for feeding infants. From the age of 6 months, the advice is for children to begin eating safe and adequate complementary foods while continuing to breastfeed for up to 2 years and beyond. Such a protracted period of breastfeeding may force mothers to express milk with a breast pump so that the milk is later available for consumption by their infants.

Expressing breastmilk may be useful in a number of different situations, in particular to enable a mother to initiate or continue breastfeeding. For example, expressed milk may be kept for a baby when their mother is separated from the baby. After milk expression, different steps may be taken to maintain the milk quality depending on the mother's situation. The process between milk expression and consumption tends to be referred to as "milk management", and this is fundamental to maintain milk quality, its nutrition properties, taste and smell.

Several guidelines have been established concerning the maximum amount of time that milk should be kept at room temperature, in the refrigerator or in the freezer. However, many parents have been found to be uncertain concerning these guidelines, particularly when the milk has been stored under different conditions, including when only temporarily storing the milk under a particular condition. The guidelines tend not to address such circumstances in which milk is stored under different conditions.

When preparing to feed their infant, caregivers and/or parents tend to face the question of which quantity or quantities of milk should be removed from their reserve of stored milk. This may present a challenge as, on the one hand, they wish to provide the freshest milk to their baby and, on the other hand, they wish to minimize the risk of milk being wasted due to expiring and having to be thrown away.

Additionally, account has to be taken of the amount of milk that comes in and out of the different storage locations, such as the refrigerator and the freezer.

In order to simplify this choice, parents tend to choose one of the following methods in which they only take one parameter into account: "first in first out" and "last in first out".

According to the "first in first out" principle, milk entering the reserve first is used first to feed the infant. According to the "last in first out" principle, milk entering the reserve last is used first to feed the infant.

Whilst application of the "last in first out" principle can assist to increase the quality of the milk being fed to the infant, it can nonetheless increase the risk of stored milk expiring.

Whilst application of the "first in first out" principle can assist to decrease the risk of stored milk expiring, the overall quality of the feeds may be lower because the quality of, e.g. nutrient amount in, the milk tends to decrease over time.

In addition to this, the hormonal composition of breast milk may change significantly over the course of the day. For example, levels of cortisol - a hormone that promotes alertness - are three times higher in morning milk than in evening milk. Melatonin, which promotes sleep and digestion, can barely be detected in daytime milk, but rises in the evening and peaks around midnight. Such compositional changes can present further milk management challenges.

### SUMMARY OF THE INVENTION

The invention is defined by the independent claims. The dependent claims define advantageous embodiments.

According to examples in accordance with an aspect of the invention there is provided a computer implemented method for communicating an infant milk management recommendation to a user, the method comprising: monitoring, over time, data indicative of a temperature of each quantity of milk in a reserve of infant milk; determining the infant milk management recommendation at least partly based on said data monitored over time; and communicating the infant milk management recommendation to the user.

Thus, the infant milk management recommendation, for example an indication of an order in which quantities of the milk, e.g. expressed breast milk, are to be fed to an infant, is based on the temperature experienced over time by each quantity of milk.

The freshness, shelf-life etc. of the quantities of milk may be more reliably accounted for in the determination of the recommendation than, for example, a scenario in which a recommendation is based on a single temperature associated with a current storage location in which the milk is stored.

In some embodiments, the data comprises temperature sensor data generated by at least one temperature sensor.

In some embodiments, a temperature sensor is associated with each said quantity of milk so as to generate temperature sensor data indicative of a temperature of the respective quantity of milk associated with the temperature sensor.

This can be implemented, for example, by each temperature sensor being arrangable on and/or in an infant milk container so as to generate temperature sensor data indicative of a temperature of the quantity of milk contained in that infant milk container.

In such embodiments, the temperature of the quantity of milk can be straightforwardly monitored over time while the infant milk container is in and being moved between different storage locations.

In some embodiments, such a temperature sensor is included in an infant milk container attachment together with a communication system arranged to communicate the temperature sensor data indicative of a temperature of the milk contained in the infant milk container to a processing system configured to implement the method.

Alternatively or additionally, the data comprises storage location data indicative of the temperature of each quantity of milk in the reserve of infant milk.

In such embodiments, the storage location data may comprise an indicator of the presence of each said quantity of milk in a freezer, refrigerator or in an ambient storage location.

The freezer may be at -20°C, the refrigerator may be at 5°C, and the ambient storage location may be at 20°C.

Thus, by monitoring the storage location data over time, an indication of the temperature of each quantity of milk in the reserve of infant milk may correspondingly be monitored over time.

The storage location data may, for example, be detected by a sensor arranged to detect the presence of each said quantity in the freezer, refrigerator or in the ambient storage location.

The user to whom the recommendation is communicated may be one or more of a mother of an infant whose expressed milk is stored in the reserve of infant milk, a carer of the infant, and a healthcare provider, such as a nurse, midwife and/or paediatrician.

In some embodiments, the method further comprises receiving timestamp data indicating when each said quantity of milk was expressed, with the determining the infant milk management recommendation being partly based on the timestamp data.

Thus, the infant milk management recommendation, for example an indication of an order in which quantities of the milk are to be fed to an infant, can be based on when the milk was expressed and the data monitored over time. The latter can indicate the temperature(s) to which the expressed milk has been exposed after being expressed.

The timestamp data may comprise a date and/or a time of expression.

In some embodiments, the monitoring over time may begin from the date and the time defined by the timestamp data.

In embodiments in which the timestamp data comprises the date and/or time of expression, the determining the infant milk management recommendation may comprise calculating a quality score based on the time elapsed since the date and/or time of expression and the data indicative of the temperature of the expressed milk monitored overtime.

Alternatively or additionally, the time of expression may be used to determine a time-of-day value indicative of whether the milk was expressed during the day or during the night.

In such embodiments, the method may further comprise receiving a next feed indicator of whether milk of the reserve of infant milk is to be next used for a night feed or a day feed, with the determining the infant milk management recommendation being partly based on the time-of-day value and the next feed indicator.

For example, if the time-of-day value is indicative of the milk being expressed during the night, the infant milk management recommendation may indicate next-to-be-used quantity or quantities of milk which was or were expressed during the night.

In this way, the infant may be fed with milk having a hormonal composition appropriate for the time of day at which the infant is being fed.

In some embodiments, the method further comprises receiving an outcome value indicative of an outcome of previous management of the reserve of infant milk, with the determining the infant milk management recommendation being partly based on the outcome value.

Thus, the outcome of the previous management, for example management arising from a previous infant milk management recommendation, may be used as a (partial) basis upon which to determine a contemporaneous infant milk management recommendation. In this way, a reinforcement learning approach may be employed in the determining of the infant milk management recommendation.

In some embodiments, the outcome comprises one or more of: an expiry date of infant milk having elapsed without the infant milk having been fed to an infant; and a measure of quality of a quantity of milk previously recommended for feeding to an infant.

The measure of quality may, for example, be the quality score described above.

In some embodiments, the determining comprises using a reinforcement learning algorithm, such as a model-free reinforcement learning algorithm.

The reinforcement learning algorithm may, for example, operate to increase a number of points, whereby points are earned according to how high the measure of quality is above a threshold, and points are deducted if the measure of quality decreases below the threshold and/or if the expiry date elapses without the infant milk having been fed to the infant.

The model-free reinforcement learning algorithm may, for example, use so-called Q-learning.

In some embodiments, the method further comprises receiving a user preference value indicative of the user's preference for managing the reserve of infant milk, with the determining the infant milk management recommendation being partly based on the user preference value.

The user preference value may be indicative of the user's preference for either minimising the risk of milk spoilage or maximising freshness of milk taken from the reserve of infant milk to feed to an infant.

The user may thus set a preference related to milk quality and milk spoilage, for example the user may decide to prioritize milk quality over minimising the risk of milk spoilage.

For instance, the determining the infant milk management recommendation may comprise, responsive to the user preference value being for maximising milk quality, determining next-to-be-used milk quantity or quantities having the highest quality score, subject to the quality score being at or above a threshold set to ensure that the milk is safe to consume.

Alternatively or additionally, the user preference value may be indicative of a maximum storage capacity of one or more storage locations.

For example, the user preference value may be indicative of a maximum storage capacity of the refrigerator and/or the freezer, e.g. the user can input that the freezer can fit a maximum amount of 10 infant milk containers.

In some embodiments, the infant milk management recommendation comprises an indication of an order in which quantities of the milk are to be fed to an infant.

In some embodiments, the communicating comprises controlling one or more user interfaces to indicate next-to-be-used quantity or quantities of milk of the reserve of infant milk.

In some embodiments, the one or more user interfaces includes a refrigerator display and/or a freezer display mountable on a refrigerator and/or freezer door.

Alternatively or additionally, the one or more user interfaces may comprise an indicator associated with each said quantity of milk. In such embodiments, the communicating comprises selectively controlling the indicator or indicators of the next-to-be-used quantity or quantities to distinguish them from a remainder of the reserve of infant milk.

Any suitable indicator can be used to distinguish the next-to-be-used quantity or quantities from the remainder of the reserve of infant milk, such as a light emitting diode.

Such a light emitting diode may, for example, illuminate so as to assist the user to identify the next-to-be-used quantity or quantities but not illuminate for the remainder of the reserve of infant milk.

In some embodiments, the method further comprises receiving an amount of milk in each said quantity of milk of the reserve of infant milk, with the determining said infant milk management recommendation being partly based on said amount of milk.

The amount of milk may be, for example, a volume and/or a mass of each said quantity of milk.

The amount of milk may, for example, be received from a scale on which a container containing the respective quantity of milk is placed to measure the amount of milk. In such embodiments, the method may include converting, using a density of the milk, a mass of the milk into a volume of the milk.

In some embodiments, the method further comprises receiving an amount of milk to be fed to an infant.

Such an amount of milk to be fed may, for example, be an amount of milk to be fed to the infant in a next feed, or an amount of milk to be fed to an infant over a given time period, for example while the infant is attending their childcare setting.

In such embodiments, the determining the infant milk management recommendation may comprise determining next-to-be-used quantity or quantities of milk of the reserve of infant milk based on the amount of milk in each said quantity and the amount of milk to be fed to the infant.

In such embodiments, the indicated next-to-be-used quantity or quantities of milk of the reserve of infant milk may correspond to an amount of milk which equals or exceeds the amount of milk to be fed to the infant.

In some embodiments, the infant milk management recommendation comprises a recommendation of a storage temperature condition for storing one or more further milk quantities to be added to the reserve of infant milk.

Alternatively or additionally, the infant milk management recommendation comprises a recommendation of a change to a storage temperature condition of at least one milk quantity in the reserve of infant milk.

Thus, the method may enable recommendation of where each quantity of milk is most appropriately stored, in the case of a further milk quantity to be added to the reserve, and/or in the case of an existing quantity of milk in the reserve.

For example, based on the milk quantity or quantities stored at a refrigerator temperature and stored at a freezer temperature, as well as in some embodiments a daily consumption of milk, the recommendation may include a recommendation of a storage temperature condition, e.g. refrigerator or freezer, for the further milk quantity to be added, and/or a recommendation of a change of storage temperature condition, e.g. freezer to refrigerator, of at least one milk quantity already in the reserve of infant milk.

According to another aspect there is provided a computer program comprising computer program code which, when executed on a computing device having a processing system, causes the processing system to perform all of the steps of the method according to any of the embodiments described herein.

According to yet another aspect there is provided an infant milk container attachment comprising: a temperature sensor arrangeable on and/or in an infant milk container; and a communication system adapted to communicate temperature sensor data from the temperature sensor to a processing system so as to enable the temperature sensor data to be monitored over time.

The infant milk container attachment can be configured in various ways in order to facilitate monitoring of the temperature sensor data over time.

The temperature sensor may be configured to collect the temperature sensor data at a sample rate, and communicate the collected temperature sensor data periodically to the processing system.

In some embodiments, the collected temperature sensor data is communicated at a lower frequency than the sample rate.

This may assist to reduce power consumption, e.g. so as to preserve the operating lifetime of a battery powering the infant milk container attachment.

For example, the sample rate may be every minute, and the collected temperature sensor data may be communicated 10 times per day. In such an example, communicating the temperature sensor data wirelessly, e.g. via Bluetooth^{®}, can mean that the operating lifetime of the battery is around 10 months.

Alternatively or additionally, the sampling and/or the communicating may be based on the sensed temperature conditions.

For example, a variable sample rate may vary in accordance with the sensed temperature conditions.

For instance, sampling may be triggered by a change in the temperature.

In some embodiments, the temperature sensor data may only be communicated in response to a specific event, for example when the infant milk container, to which the infant milk container attachment is attached, is used.

In at least some embodiments, the communication system is adapted to communicate temperature sensor data from the temperature sensor to a processing system arranged to execute the computer program code of the computer program according to embodiments described herein.

In some embodiments, the infant milk container attachment is in the form of a label affixable to the infant milk container.

In some embodiments, the infant milk container attachment also includes the indicator controllable by the processing system to indicate that the milk contained in the container is included in the next-to-be-used quantity or quantities.

For example, the communication system may be configured to receive a control signal from the processing system, with the indicator being controllable according to the control signal.

The control signal may, for example, be generated by the processing system according to the determined infant milk management recommendation.

In embodiments in which an infant milk management system comprises a plurality of the infant milk container attachments, the control signal be may selectively sent to the communication system of the infant milk container attachment(s) attached to container(s) containing the next-to-be-used quantity or quantities to distinguish them from the other infant milk container attachment(s) of the reserve of infant milk.

According to a further aspect there is provided an infant milk management system comprising a processing system configured to monitor, over time, data indicative of a temperature of each quantity of milk in a reserve of infant milk; determine said infant milk management recommendation at least partly based on said data monitored over time; and control one or more user interfaces to communicate the infant milk management recommendation to the user.

In some embodiments, the data comprises temperature sensor data generated by at least one temperature sensor.

The at least one temperature sensor may be included in the system.

Alternatively, the at least one temperature sensor may be provided, for example supplied to the user, separately from the processing system. Such a scenario may arise, for example, when the processing system is included in a user device and/or in a cloud-based server.

In some embodiments, a temperature sensor, of the at least one temperature sensor, is associable with each said quantity of milk so as to generate temperature sensor data indicative of a temperature of the respective quantity of milk associated with the temperature sensor.

In some embodiments, the system comprises one or more of the infant milk container attachment, for example one or more of the infant milk container attachment in the form of the label.

In some embodiments, the user interface controllable to communicate the infant milk management recommendation to the user is included in the system.

Alternatively, the user interface may be provided, e.g. supplied to, the user separately from the processing system.

More generally, embodiments described herein in relation to the method and computer program may be applicable to the infant milk container attachment and system, and embodiments described herein in relation to the infant milk container attachment and system may be applicable to the method and computer program.

### BRIEF DESCRIPTION OF THE DRAWINGS

Examples of the invention will now be described in detail with reference to the accompanying drawings, in which:
FIG. 1 provides a flowchart of a method according to an example;
FIG. 2 provides a block diagram of an infant milk container attachment according to a first example;
FIG. 3 provides a block diagram of a system according to an example;
FIG. 4 provides a block diagram of a system according to another example;
FIG. 5 provides a flowchart of a method according to another example;
FIG. 6 provides graphs showing the decline in quality of infant milk over time at different storage conditions;
FIG. 7 provides graphs showing the decline in quality of infant milk in each of four quantities of frozen milk of an infant milk reserve, together with an ordering according to the "first in first out" principle;
FIG. 8 shows the same graphs as shown in FIG. 7, together with an ordering according to the "last in first out" principle;
FIG. 9 shows the same graphs as shown in FIGs. 7 and 8, together with an ordering recommended using a method according to an example of the present disclosure;
FIG. 10 provides a graph of nutrient quantity in milk vs time;
FIG. 11 provides a flowchart of a method according to yet another example;
FIG. 12 provides a flowchart of a method according to a further example;
FIG. 13 shows an infant milk container attachment according to a second example;
FIG. 14 shows the infant milk container attachment shown in FIG. 13 attached to an infant milk container in the form of a bottle;
FIG. 15 shows an infant milk container attachment according to a third example;
FIG. 16 shows the infant milk container attachment shown in FIG. 15 attached to an infant milk container in the form of a bag;
FIG. 17 shows an infant milk container attachment according to a fourth example;
FIG. 18 shows an infant milk container attachment according to a fifth example;
FIG. 19 shows an infant milk container attachment according to a sixth example;
FIGs. 20A and 20B show different indicator states of the infant milk container attachment shown in FIG. 17 attached to an infant milk container in the form of a bottle;
FIGs. 21A and 21B show different indicator states of the infant milk container attachment shown in FIG. 19 attached to an infant milk container in the form of a bag;
FIG. 22 shows an infant milk container attachment according to a seventh example;
FIGs. 23A and 23B show different indicator states of an infant milk container attachment according to an eighth example;
FIG. 24 shows a first example of an attachment member for affixing the infant milk container attachment shown in FIG. 22 to an infant milk container in the form of a bag;
FIG. 25 shows the infant milk container attachment shown in FIG. 22 attached to an infant milk container in the form of a bag using the attachment member shown in FIG. 24;
FIG. 26 shows a second example of an attachment member for affixing the infant milk container attachment shown in FIG. 22 to an infant milk container in the form of a bottle;
FIG. 27 shows the attachment member shown in FIG. 26 with the infant milk container attachment shown in FIG. 22 attached thereto;
FIG. 28 shows the infant milk container attachment shown in FIG. 22 attached to an infant milk container in the form of a bottle using the attachment member shown in FIG. 26;
FIG. 29 shows a refrigerator display and/or a freezer display mountable on a refrigerator and/or freezer door;
FIG. 30 shows a scale on which an infant milk container containing a quantity of milk is placed to measure an amount of milk in the container;
FIG. 31 shows milk container labels according to an example;
FIG. 32 shows one of the milk container labels shown in FIG. 31 affixed to an infant milk container in the form of a bottle;
FIG. 33 shows one of the milk container labels shown in FIG. 31 affixed to an infant milk container in the form of a bag; and
FIGs. 34 to 36 provide screenshots of a smartphone application according to an example.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a computer implemented method for communicating an infant milk management recommendation to a user. The method comprises monitoring, over time, data indicative of a temperature of each quantity of milk in a reserve of infant milk. The infant milk management recommendation is determined at least partly based on said data monitored over time, and the recommendation is communicated to the user. Further provided is a computer program for implementing the method, an infant milk container attachment, and an infant milk management system.

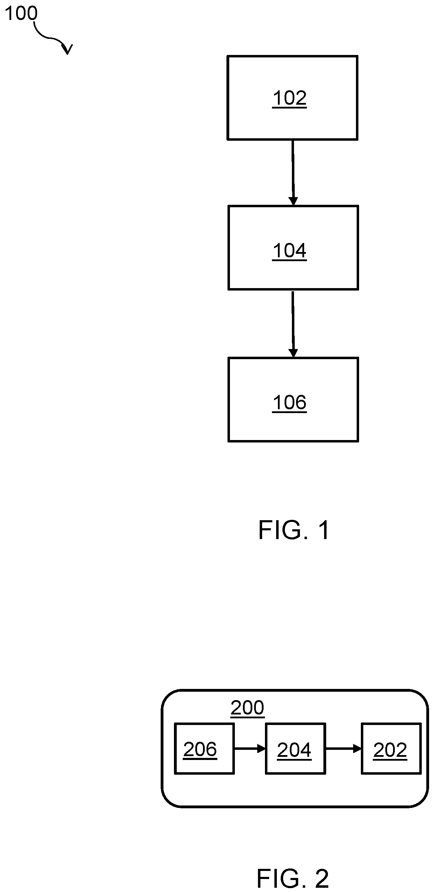

FIG. 1 provides a flowchart of a method 100 according to an example. The method 100 comprises monitoring 102, over time, data indicative of a temperature of each quantity of milk in a reserve of infant milk.

The method 100 further comprises determining 104 an infant milk management recommendation at least partly based on the data monitored over time, and communicating 106 the infant milk management recommendation to a user.

The user may be one or more of a mother of an infant whose expressed milk is stored in the reserve of infant milk, a carer of the infant, and a healthcare provider, such as a nurse, midwife and/or paediatrician.

The freshness, shelf-life etc. of the quantities of milk may be more reliably accounted for in the determination 104 of the recommendation based on the data monitored over time than, for example, a scenario in which a recommendation is based on a single temperature associated with a current storage location in which the milk is stored.

Various data types are contemplated for the data indicative of the temperature of each quantity of milk in a reserve of infant milk. In some embodiments, the data comprises storage location data indicative of the temperature of each quantity of milk in the reserve of infant milk. In such embodiments, the storage location data may comprise an indicator of the presence of each said quantity of milk in a freezer, refrigerator or in an ambient storage location. The freezer may be at -20°C, the refrigerator may be at 5°C, and the ambient storage location may be at 20°C. Thus, by monitoring the storage location data over time, an indication of the temperature of each quantity of milk in the reserve of infant milk may correspondingly be monitored over time.

The storage location data may, for example, be detected by a sensor arranged to detect the presence of each said quantity in the freezer, refrigerator or in the ambient storage location.

Alternatively or additionally, the data comprises temperature sensor data generated by at least one temperature sensor.

In some embodiments, a temperature sensor is associated with each said quantity of milk so as to generate temperature sensor data indicative of a temperature of the respective quantity of milk associated with the temperature sensor.

This can be implemented, for example, by each temperature sensor being arrangeable on and/or in an infant milk container so as to generate temperature sensor data indicative of a temperature of the quantity of milk contained in that infant milk container.

In such embodiments, the temperature of the quantity of milk can be straightforwardly monitored over time while the infant milk container is in and being moved between different storage locations.

The term "infant milk management" as used herein may refer to the process of handling and/or storing milk between milk expression and consumption by an infant.

In some embodiments, the infant milk management recommendation comprises an indication of an order in which quantities of the milk are to be fed to the infant.

Alternatively or additionally, the infant milk management recommendation comprises a recommendation of a storage temperature condition for storing one or more further milk quantities to be added to the reserve of infant milk.

Alternatively or additionally, the infant milk management recommendation comprises a recommendation of a change to a storage temperature condition of at least one milk quantity in the reserve of infant milk.

Thus, the method 100 may enable recommendation of which quantity/quantities of milk should be fed next to the infant, and where each quantity of milk is most appropriately stored, in the case of a further milk quantity to be added to the reserve, and/or in the case of an existing quantity of milk in the reserve.

In some embodiments, the communicating 106 comprises controlling one or more user interfaces to indicate next-to-be-used quantity or quantities of milk of the reserve of infant milk.

FIG. 2 provides a block diagram of an infant milk container attachment 200 according to an example. In some embodiments, the infant milk container attachment 200 is in the form of a label affixable to an infant milk container.

In at least some embodiments, the infant milk container attachment 200 comprises an indicator 202, such as a visual indicator 202 controllable to communicate infant milk management information.

In some non-limiting examples, the indicator 202 comprises an electronic-ink display for displaying the infant milk management information, for example to communicate the infant milk management recommendation. Alternatively or additionally, the indicator 202 may include a light emitting diode.

Such an electronic-ink display may be operable at temperatures as low as -40°C. Hence such an electronic-ink display may be suitable for communicating the infant milk management information, e.g. the infant milk management recommendation, when the infant milk container attachment 200 is located in a typical household freezer whose temperature is -20°C.

More generally, the infant milk container attachment 200 may be powerable by one or more batteries. It is noted that batteries may be operable to power the infant milk container attachment 200 at temperatures as low as -50°C, albeit with an efficiency which decreases with decreasing temperature.

In some embodiments, the infant milk container attachment 200, e.g. label, includes one or more buttons for user control.

In some embodiments, the one or more buttons includes a reset button actuatable to refresh the indicator 202 and/or remove the respective quantity of milk associated with the infant milk container attachment 200 from the reserve of infant milk.

The infant milk container attachment 200, e.g. in the form of a label, is preferably a stand-alone unit attachable to an infant milk container, for example using a click-on system or any other suitable attachment mechanism such as tape, Velcro, etc. In the case of milk bags, a clip can be used, as will be described in more detail herein below.

In some embodiments, such as that shown in FIG. 2, the infant milk container attachment 200 includes a communication system 204 configured to receive a control signal from a processing system (not visible in FIG. 2), with the indicator 202 being controllable according to the control signal.

The communication system 204 is preferably a wireless communication system 204.

Any suitable wireless communications protocol can be employed in such a wireless communication system 204, such as near-field communication (NFC), Bluetooth^{®}, Wi-Fi, etc.

The control signal may, for example, be generated by the processing system according to the determined infant milk management recommendation.

In some embodiments, the indicator 202 is controllable to indicate that the milk contained in the container to which the infant milk container attachment 200 is attached is included in the next-to-be-used quantity or quantities.

In embodiments in which an infant milk management system comprises a plurality of the infant milk container attachments 200, the control signal be may selectively sent to the communication system of the infant milk container attachment(s) 200 attached to the container(s) containing the next-to-be-used quantity or quantities to distinguish them from the other infant milk container attachment(s) 200.

Any suitable indicator 202 can be used to distinguish the next-to-be-used quantity or quantities them from a remainder of the reserve of infant milk, such as the above-mentioned light emitting diode and/or electronic-ink display.

Such a light emitting diode may, for example, illuminate so as to assist the user to identify the next-to-be-used quantity or quantities but not illuminate for the remainder of the reserve of infant milk. In the case of the electronic-ink display, a symbol may appear on the electronic-ink display(s) associated with the next-to-be-used quantity or quantities but not on the display(s) associated with the remainder of the reserve of infant milk.

In such embodiments the infant milk container attachment 200, e.g. in the form of a label attachable to an infant milk container, dynamically displays via the indicator 202 whether the infant milk container to which the infant milk container attachment 200 is attached is the recommended milk container to use among a plurality of other milk containers for the next feeding of the infant.

As an alternative or in addition to the indicator 202, the infant milk container attachment 200 can include a temperature sensor 206.

In such embodiments, the communication system 204 may be arranged to communicate temperature sensor data indicative of a temperature of the milk contained in the infant milk container to a processing system (not visible in FIG. 2) so as to enable the temperature sensor data to be monitored over time.

The infant milk container attachment 200 may continuously, or at least periodically, upload the temperature sensor data, e.g. to a database (not visible in FIG. 2), such as a database included in a cloud-based server and/or in a user device (also not visible in FIG. 2). Examples of the user device include a smartphone, tablet computer and/or personal computer.

The temperature sensor 206 may be arrangeable to obtain the temperature of the environment in which the respective quantity of milk, contained in the infant milk container to which the infant milk container 200 is attached, is located or the temperature of the milk itself.

The temperature sensor data may be primarily used to estimate the location, and change in location, of the respective infant milk container to which the infant milk container attachment 200 is attached. The location may include an ambient temperature location, refrigerator, and freezer.

In some embodiments, the determining 104 comprises using the temperature sensor data to determine 104 a position of one or more quantities of milk of the reserve of infant milk within a refrigerator.

A position in a door of the refrigerator may, for example, be determinable by the temperature sensor data being indicative of a difference in temperature within the refrigerator and/or an effect of opening the door of the refrigerator.

In such embodiments, the infant milk management recommendation may include advice to check the location of one or more of said quantities determined to be positioned in the door and/or an instruction to move the one of more of said quantities from the door to an interior shelf of the refrigerator.

This may assist the user to comply with advice not to store milk in the refrigerator's door.

Alternatively or additionally, an estimation of milk quality may be made using the thus determined position of the one or more quantities of milk in the refrigerator, and/or more generally the data indicative of the temperature of the one or more quantities of milk, as will be explained in more detail herein below.

In some embodiments, the temperature sensor 206 is configured to continue to generate temperature sensor data indicative of the respective quantity of milk in the infant milk container to which the infant milk container attachment 200 is attached following removal of the infant milk container from its storage location and preparation for the infant. Thus, the quantity milk may be monitored up to, and in some cases during, its consumption by the infant.

In some embodiments, the infant milk container attachment 200 includes an optical sensor (not visible), for example a UV and/or visible light sensor.

Such an optical sensor may enable sensing a level of exposure to ultraviolet and/or blue light, which may be harmful for the milk.

In some embodiments, the method 100 may include receiving a sensed level of exposure to ultraviolet and/or blue light, with the determining the recommendation being based on the sensed level of exposure.

In some embodiments, data from the optical sensor can be included in, for example so as to improve, the data indicative of the temperature of the milk because the data from the optical sensor can be indicative of the storage location. This is because of the darkness inside the refrigerator or freezer when its door is closed.

In at least some embodiments, the communication system 204 also included in the infant milk container attachment 200 is adapted to communicate temperature sensor data from the temperature sensor 206 to a processing system arranged to implement the method 100 according to embodiments described herein.

In embodiments, such as that shown in FIG. 2, in which the infant milk container attachment 200 includes the indicator 202 and the temperature sensor 206, the communication system 204 may be arranged to communicate the temperature sensor data to the processing system, and receive the control signal from the processing system to control the indicator 202 should the infant milk container attachment 200 be attached to a container containing one of the next-to-be-used quantity or quantities.

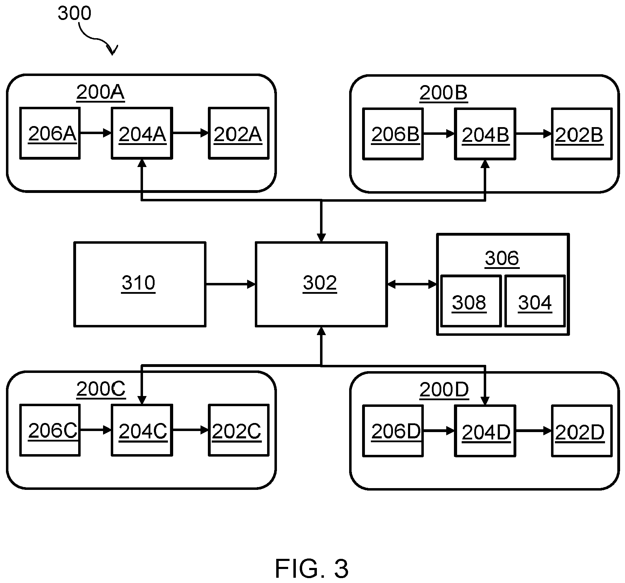

FIG. 3 provides a block diagram of an infant milk management system 300 according to an example. The infant milk management system 300 comprises a processing system 302 configured to implement the method 100 according to embodiments described herein.

In particular, the processing system 302 is configured to monitor 102, over time, data indicative of a temperature of each quantity of milk in a reserve of infant milk, determine 104 the infant milk management recommendation at least partly based on said data monitored over time, and control one or more user interfaces to communicate 106 the infant milk management recommendation to the user.

In some embodiments, such as that shown in FIG. 3, the data comprises temperature sensor data generated by at least one temperature sensor 206A, 206B, 206C, 206D.

Whilst four temperature sensors 206A, 206B, 206C, 206D are shown in FIG. 3, there may be any number of temperature sensors 206A, 206B, 206C, 206D provided that data indicative of the temperature of each quantity of milk in the reserve of infant milk can be provided to the processing system 302.

Each temperature sensor, of the at least one temperature sensor 206A, 206B, 206C, 206D, may be associable with each said quantity of milk so as to generate temperature sensor data indicative of a temperature of the respective quantity of milk associated with the temperature sensor.

In some embodiments, such as that shown in FIG. 3, the system 300 comprises one or more of the infant milk container attachment 200A, 200B, 200C, 200D, for example one or more of the infant milk container attachment 200A, 200B, 200C, 200D in the form of the label affixable to the respective infant milk container.

In such embodiments, the processing system 302 may receive the temperature sensor data via communication systems 204A, 204B, 204C, 204D included in each of the infant milk container attachments 200A, 200B, 200C, 200D.

The one or more user interfaces controlled to communicate the infant milk management recommendation may, in some embodiments, include a user output interface 304 included in a user device 306.

Such a user device 306 can, for example, be one or more selected from a smartphone, tablet computer, and personal computer. In such examples, the user output interface 304 may include a display functionality of a touchscreen included in the smartphone, tablet computer, and/or personal computer.

Controlling such a user output interface 304 to communicate the infant milk management recommendation, as well as other pertinent information relating to the reserve of infant milk will be described in more detail herein below.

The system 300 may also include one or more user input interfaces 308 configured to enable the user to input data relevant to the reserve of infant milk. Various examples of such user input data are described herein.

In some embodiments, such as that shown in FIG. 3, the user input interface 308 may be included in the user device 306. For example, the user input interface 308 may include touchscreen functionality of the touchscreen of the smartphone, tablet computer, and/or personal computer.

In some embodiments, such as that shown in FIG. 3, the one or more user interfaces include the indicators 202A, 202B, 202C, 202D included in each of the infant milk container attachments 200A, 200B, 200C, 200D.

In such embodiments, the processing system 302 may be configured to selectively control the indicator(s) 202A, 202B, 202C, 202D, e.g. by sending a control signal receivable via one or more of the communication systems 204A, 204B, 204C, 204D, to the infant milk container attachment(s) 200A, 200B, 200C, 200D attached to the container(s) containing the next-to-be-used quantity or quantities to distinguish them from the other infant milk container attachment(s) 200A, 200B, 200C, 200D of a remainder of the reserve of infant milk, as previously described.

In some embodiments, and whilst not shown as a step in FIG. 1, the method 100 further comprises receiving an amount of milk in each said quantity of milk of the reserve of infant milk. In such embodiments, the determining 104 the infant milk management recommendation may be partly based on the amount of milk. The amount of milk may be, for example, a volume and/or a mass of each said quantity of milk.

In some embodiments, and referring again to FIG. 3, the amount of milk may, for example, be received from a milk amount input device 310, such as a scale on which a container containing the respective quantity of milk is placed to measure the amount of milk.

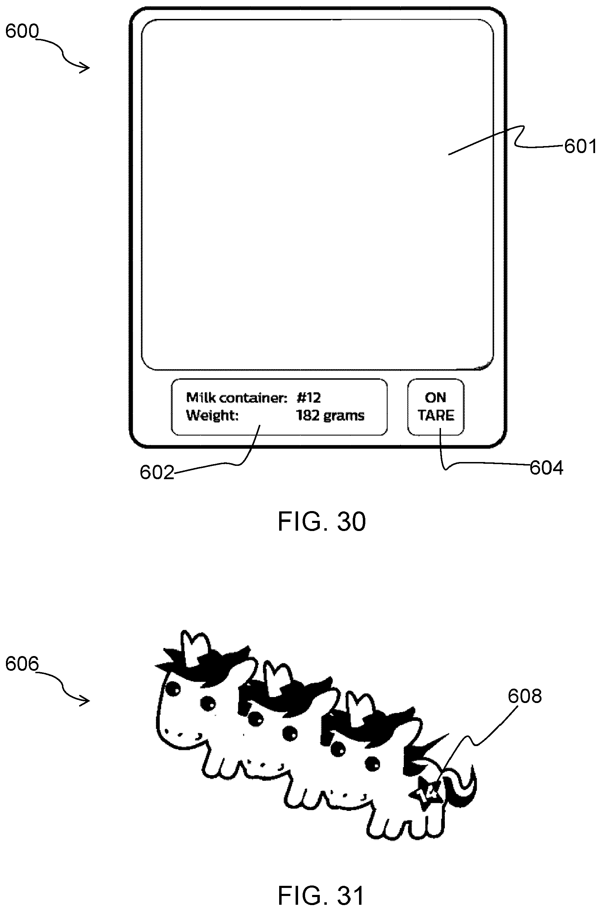

Embodiments relating to such a scale will be described in more detail herein below with reference to FIG. 30.

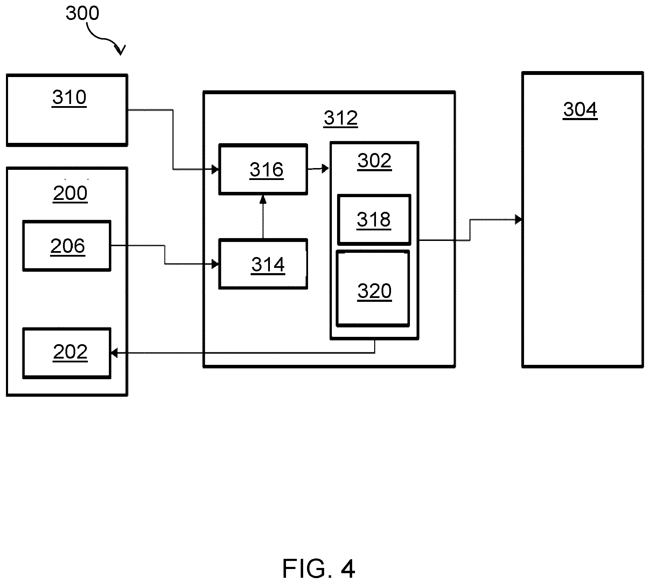

FIG. 4 shows a system 300 according to another example. Similarly to the example shown in FIG. 3, the system 300 shown in FIG. 4 comprises a processing system 302 configured to monitor 102, over time, data indicative of a temperature of each quantity of milk in a reserve of infant milk, determine 104 the infant milk management recommendation at least partly based on said data monitored over time, and control one or more user interfaces to communicate 106 the infant milk management recommendation to the user.

In the non-limiting example shown in FIG. 4, the processing system 302 is included in a cloud-based server 312. The one or more user interfaces 202, 304 may be in communication with the processing system 302 via the cloud-based server 312 so as to enable the processing system 302 to control the one or more user interface 202, 304 to communicate the infant milk management recommendation to the user. The latter is schematically depicted in FIG. 4 by the arrow between the processing system 302 and the indicator 202 of the infant milk container attachment 200, and the arrow between the processing system 302 and the user output device 304, e.g. included in a user device 306, such as a user device selected from a smartphone, a tablet computer, and/or a personal computer.

Such a user device may be loaded with an application configured to enable the user output device 304, e.g. the display functionality of a touchscreen, to communicate the infant milk management recommendation to the user.

In some embodiments, the user output device 304 is configured to communicate: the name of the infant to whom each said quantity of milk is to be fed; the date of expression of each said quantity of milk; the time of expression of each said quantity of milk; the amount, e.g. volume and/or mass, of each said quantity of milk; and the indication on the next-to-be used quantity of milk.

Alternatively or in addition to the above-listed user outputs, the user output device 304 may be configured to communicate: the location of each said quantity of milk, e.g. the location of the next-to-used quantity of milk, such as the refrigerator or freezer; an indication of the milk quality; and a milk expiry indication should the processing system determine, e.g. based on the time-monitored data, that the respective quantity of milk has expired.

Similarly to the example shown in FIG. 3, the data monitored over time by the processing system 302 shown in FIG. 4 comprises temperature sensor data generated by at least one temperature sensor 206. The latter is included with the indicator 202 in the infant milk container attachment 200, e.g. in the form of a label, as previously described in relation to FIG. 3.

The monitoring over time may make use of a timer module 314 included in the cloud-based server 312, in the infant milk container attachment 200, and/or in the processing system 302. The timer module 314 may, for example, track an amount of time during which each said quantity is at a particular temperature.

The milk amount input device 310 shown in FIGs. 3 and 4 may comprise a scale and/or a breast pump arrangement configured to communicate the amount, e.g. volume and/or mass, of milk in each said quantity.

The milk amount input device 310 may enable the amount, e.g. volume and/or mass, of the milk in each said quantity to be inputted from a sensor.

Alternatively or additionally, the amount of milk may be user inputted, e.g. via the user input interface 308.

In a non-limiting example, the amount of milk may be user-inputted via the user input interface 308 of a user device 306, e.g. comprising a smartphone, running an application comprising a field for the user to enter the amount of milk.

In some embodiments, the milk amount input device 310 comprises a breast pump arrangement comprising a milk volume sensor, with the breast pump arrangement being arranged to communicate, e.g. wirelessly communicate, the sensed amount of milk to the processing system 302.

The breast pump arrangement may, for example, comprise a wearable breast pump.

In embodiments in which the milk amount input device 310 comprises the scale, a breast pump arrangement may be configured to transmit the amount of milk from the scale to the processing system 302, e.g. via the cloud-based server 312 as shown in FIG. 4. Alternatively or additionally, the user device 306, e.g. the smartphone, table computer, and/or personal computer, may be configured to communicate the amount of milk from the scale to the processing system 302.

In some embodiments, a milk amount sensor, e.g. a milk volume sensor, is included, e.g. integrated into, the infant milk container attachment 200, and configured to sense the amount of milk contained in the infant milk container.

For example, the infant milk container attachment 200, e.g. label, may include a time of flight sensor arranged to determine a distance to a surface of the milk contained in the infant milk container.

Such a sensor may, for instance, be placed on a container-facing side of the infant milk container attachment 200, e.g. label, at the lid of the infant milk container.

A volume of the milk within the infant milk container may be estimated based on a value from the sensor and parameters of the infant milk container.

Alternative sensor types can also be contemplated for inclusion in the infant milk container attachment 200, such as an optical sensor, a force sensor, e.g. a weighing scale, an ultrasonic sensor, etc.

The amount of milk in each said quantity in the reserve of infant milk together with the time-monitored data indicative of the temperature of each quantity of milk may be stored in a database 316. In the non-limiting example shown in FIG. 4, the database 316 is included in the cloud-based server 312. Alternatively or additionally, the database 316 can be included in the user device 306.

The processing system 302 may receive the time-monitored data and, in at least some embodiments, the amount of milk in each said quantity from the database 316 in order to determine 104 the milk management recommendation.

In some embodiments, such as that shown in FIG. 4, the processing system 302 comprises a milk localization unit 318 configured to determine 104 a recommendation of a storage temperature condition for storing one or more further milk quantities to be added to the reserve of infant milk, and/or a recommendation of a change to a storage temperature condition of at least one milk quantity in the reserve of infant milk.

Alternatively or additionally, the processing system 302 may comprise a milk quality ordering unit 320 configured to determine 104 an indication of an order in which quantities of the milk are to be fed to an infant.

In some embodiments, a breast pump arrangement is connected to the user device 306, e.g. a smartphone, such that milk management information about the expressed milk is available via the user output interface 304.

In such embodiments, the infant milk container attachment 200 may be configured to receive the milk management information from the user device 306.

For example, the user may place the infant milk container attachment 200, e.g. label, in contact with the user device 306, e.g. smartphone, to automatically transfer the milk management information between the user device 306 and the infant milk container attachment 200.

By using the user device 306, e.g. smartphone, as an intermediary in this manner, the connectivity and processing power needed within the breast pump arrangement may be reduced, and the infant milk container attachment 200 may be used with any connected breast pump arrangement.

In another embodiment, data regarding the expression session is entered manually by the user via the user input interface 308, for example by the user completing one or more fields in an application running on the user device 306, e.g. smartphone.

Such data regarding the expression session may then be transferred from the user device 306 to the infant milk container attachment 200.

The connection with the infant milk container attachment 200 may be wireless, e.g. by NFC, as previously described.

Alternatively or additionally, a camera included in the user device 306, e.g. smartphone, phone may identify the infant milk container attachment 200 associated with a quantity of milk, for example by identifying a QR code or barcode on the infant milk container attachment 200, e.g. label.

In a non-limiting example of a use case, when the user has completed a milk expression session, a connected breast pump arrangement automatically uploads information about the milk, including milk expression time and milk volume, to the database 316 in the cloud-based server 312. The user then places an infant milk container attachment 200, e.g. in the form of an electronic milk label (EML), on the infant milk container in which the milk from the expression session is received. By attaching the EML to the infant milk container, the information of the latest uploaded milk information may be displayed via the indicator 202, e.g. display, and the milk expression information specific to this milk is assigned to this EML in the database. The processing system 302 then uses the data available to estimate which of the available milk containers to recommend and displays this information via the indicator 202 and via the user output interface 304, e.g. by a visual provided in an application being run by the user device 306, e.g. smartphone.

When a milk container is used by the user, the user may push a reset button included in, and/or associated with, the infant milk container attachment 200 to refresh the indicator 202 and remove the associated quantity of milk from the database 316.

Following receiving a notification of removal of one or more quantities of milk from the reserve, the processing system 302 may automatically update the remaining infant milk container attachments 200, e.g. to indicate which is the next-to-be-used quantity or quantities to use.

More generally, reset of the infant milk container attachment 200 such that the infant milk container attachment 200 can be associated with a further quantity of milk to be added to the reserve can be implemented via one or more of the following: actuation of a reset button included in the infant milk container attachment 200; manual entry from the user via the user input interface 308, e.g. via user-selectable option accessible via an application running on the user device 306, e.g. smartphone; automatic detection of room temperature via the temperature sensor 206 included in the infant milk container attachment 200; via a sensor, e.g. an accelerometer and/or volume sensor detecting if the infant milk container is being used for consumption so as to justify resetting of the infant milk container attachment 200; and via scanning of the infant milk container attachment with a user device 306, such as a smartphone equipped with NFC or a camera.

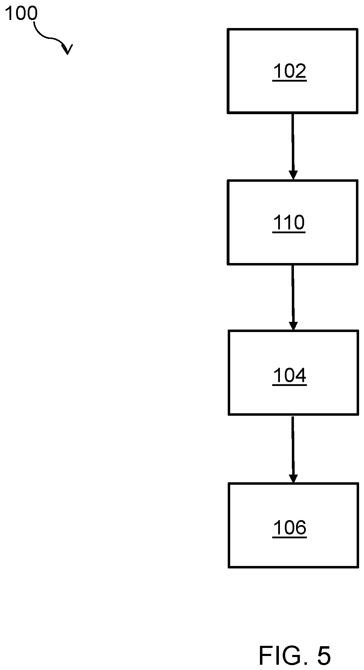

In some embodiments, such as that shown in FIG. 5, the method 100 comprises receiving 110 timestamp data indicating when each said quantity of milk was expressed, with the determining 104 the infant milk management recommendation being partly based on said timestamp data. The timestamp data may comprise a date and/or a time of expression.

In embodiments in which the timestamp data comprises the date and/or time of expression, the determining 104 the infant milk management recommendation may comprise calculating a quality score based on the time elapsed since the date and/or time of expression and the data indicative of the temperature of the expressed milk monitored overtime.

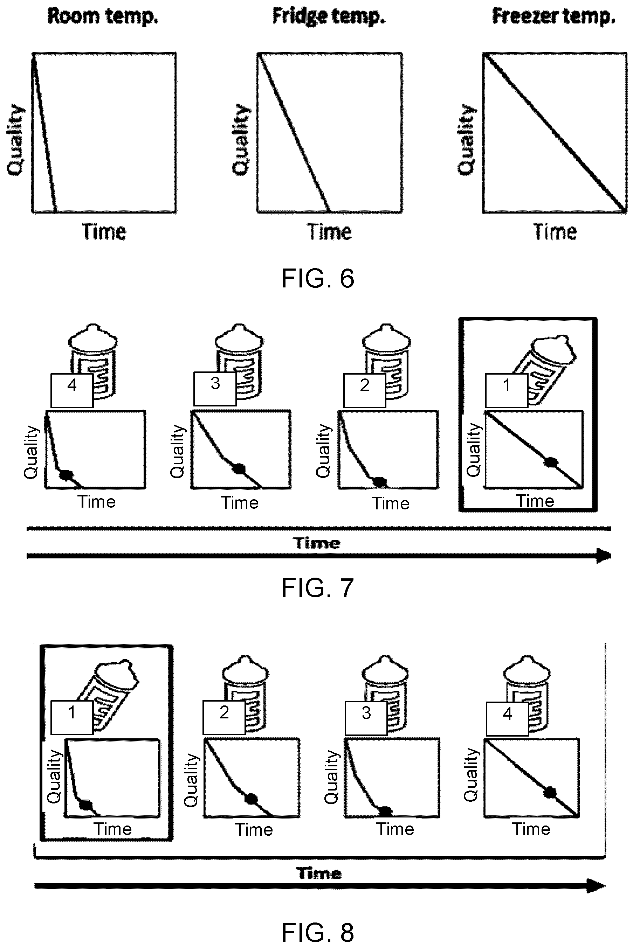

Referring to FIG. 6, the gradient of decline in milk quality with time becomes shallower as the temperature at which the milk is stored decreases. Thus, the steepest decline in milk quality with time is for milk stored at room temperature, e.g. 20°C, as shown in the left hand graph of FIG. 6, and the shallowest decline in milk quality with time is for milk stored in the freezer, e.g. at -20°C, as shown in the right hand graph of FIG. 6. The decline in milk quality with time for milk stored in the refrigerator, e.g. at 5°C, is between that of freezer- and room temperature-stored milk, as shown in the middle graph of FIG. 6.

FIGs. 7 to 9 show four milk quantities currently stored in a freezer but which have each been previously exposed to different temperatures. The far left hand graph is for a quantity initially stored at room temperature, and then stored in the freezer. The left graph of the two central graphs is for a quantity initially stored in the refrigerator, and then stored in the freezer. The right graph of the two central graphs is for a quantity initially stored at room temperature, then stored in the refrigerator, and finally stored in the freezer. The far right hand graph is for a quantity stored solely in the freezer during this period of time.

FIG. 7 provides an ordering of use of the quantities according to the "first in first out" principle, where "1" indicates the next-to-be-used quantity and "4" indicates the last to be used quantity. Thus, the quantity of the far right hand graph should be the next-to-be-used, on the basis that it was the first of the four quantities to be stored in the reserve.

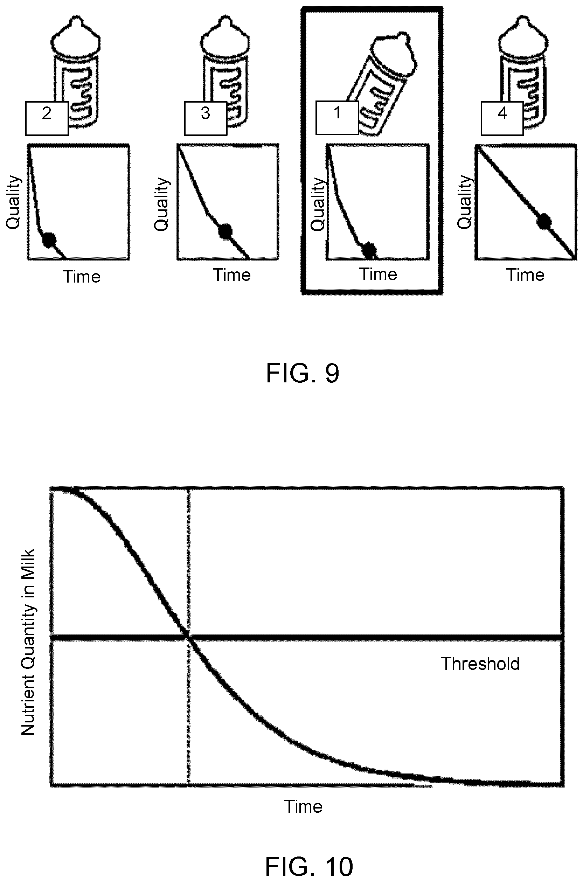

However, this quantity is not, in actual fact, the quantity most at risk of expiring without having been used to feed an infant. Referring also to the nutrient quantity threshold shown in FIG. 10, the lowest quality quantity that is most at risk of falling below an acceptable quality threshold for feeding to an infant is denoted by "2" in FIG. 7.

FIG. 8 provides an ordering of use of the quantities according to the "last in first out" principle, where again "1" indicates the next-to-be-used quantity and "4" indicates the last to be used quantity. Thus, the quantity of the far left hand graph should be the next-to-be-used, on the basis that it was the last of the four quantities to be stored in the reserve.

However, this quantity is not, in actual fact, the highest quality quantity of milk in the reserve. Rather, the highest quality quantity is denoted by "4" in FIG. 7.

Hence in spite of the "first in first out" principle seeking to minimize wastage of milk and the "last in first out" principle seeking to maximize the quality of milk fed to the infant, basing such determinations only on the current storage conditions and the date of entry into the reserve, e.g. the date of expression, may risk an undesired outcome.

However, by monitoring, over time, the data indicative of the temperature of each quantity of milk, the risk of wastage of milk may be minimized, as shown by the ordering shown in FIG. 9, or the quality of milk being fed to the infant may be maximised.

Such ordering may be made, for instance, via the above-mentioned quality score calculated based on the time elapsed since the date and/or time of expression and the data indicative of the temperature of the expressed milk monitored overtime.

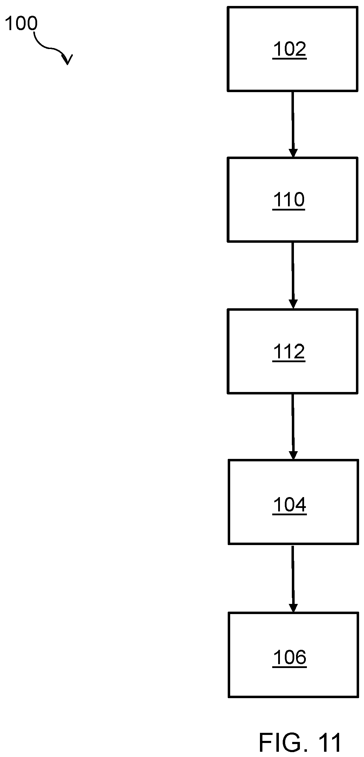

In some embodiments, such as that shown in FIG. 11, the method 100 further comprises receiving 112 a user preference value indicative of the user's preference for managing the reserve of infant milk, with the determining 104 the infant milk management recommendation being partly based on the user preference value.

The user preference value may be indicative of the user's preference for either minimising the risk of milk spoilage or maximising freshness of milk taken from the reserve of infant milk to feed to an infant.

The user may thus set a preference related to milk quality and milk spoilage, for example the user may decide to prioritize milk quality over minimising the risk of milk spoilage.

Alternatively or additionally, the user preference value may be indicative of a maximum storage capacity of one or more storage locations.

For example, the user preference value may be indicative of a maximum storage capacity of the refrigerator and/or the freezer, e.g. the user can input that the freezer can fit a maximum amount of 10 infant milk containers.

The user preference value may, for example, be entered via the one or more user input interfaces 308, for example via a user input interface 308 included in the user device 306.

In some embodiments, the determining 104 the infant milk management recommendation comprises, responsive to the user preference value being for maximising milk quality, determining next-to-be-used milk quantity or quantities having the highest quality score, subject to the quality score being at or above a threshold set to ensure that the milk is safe to consume.

Alternatively or additionally, the time of expression may be used to determine a time-of-day value indicative of whether the milk was expressed during the day or during the night.

In such embodiments, the method 100 may further comprise receiving a next feed indicator of whether milk of the reserve of infant milk is to be next used for a night feed or a day feed, with the determining 104 the infant milk management recommendation being partly based on the time-of-day value and the next feed indicator.

For example, if the time-of-day value is indicative of the milk being expressed during the night, the infant milk management recommendation may indicate next-to-be-used quantity or quantities of milk which was or were expressed during the night.

Referring again to FIG. 4, the information stored in the database 316, included in the user device 306, e.g. smartphone and/or in the cloud-based server 312, may be analyzed automatically by the processing system 302. From the time-monitored data, an estimation may be made on the milk spoiling and/or the type of milk, for example day milk vs night milk. An algorithm may then estimate which quantity/quantities of milk, e.g. which of the milk container(s), is recommended for use in the next feeding session in order to maximize milk quality and milk type, while minimizing milk spoilage.

Alternatively or additionally, the algorithm may also estimate the most optimal storage location of a milk container. Based on the available milk supply, in other words the amount of milk in each said quantity, and milk usage, the infant milk management recommendation may include advice to the user to place one or more of the quantities at a specified storage location.

For example, based on the amount of milk stored in the refrigerator and a threshold milk volume based on the daily consumption of milk, the infant milk management recommendation may include advice to place a further quantity of milk, in other words a new milk container, in the refrigerator or in the freezer; and/or to relocate one or more quantities of milk from the refrigerator to the freezer, or from the freezer to the refrigerator.

In a specific non-limiting example, the processing system 302 determines the location of each said milk quantity, e.g. milk container, from the temperature sensor data generated by the temperature sensor 206 included in the infant milk container attachment 200, e.g. label. The above-mentioned quality score may be used to describe the quality of milk, which therefore has the highest score after expression and decreases over time, based on the environment in which the milk is placed, room/ambient-, refrigerator- or freezer temperature, and the time at these specific locations.

The algorithm can be, for example, based on a decision tree. In one embodiment, the selection of the milk container is made by first selecting only the milk containers which are fit for the specific time of the day (night milk vs day milk) and then choosing the lowest score. This would mainly prevent milk spoilage.

At this point it is noted that traditional supervised machine learning methods could theoretically support parents/caregivers in managing the milk reserve, e.g. via a mobile application. However, this may not be a convenient method because of (i) the requirement for a relatively large data set from a large number of participants, e.g. mothers, to train a generalizable model; (ii) a significant amount of labeled data being required to train the model, where labelling needs to be done either by the users, e.g. via user feedback, or by an expert who may likely produce inaccurate labels in labelling without context; (iii) supervised learning being difficult to adapt/personalize, with such personalization tending to require training many models on all different pre-defined cases; and (iv) many supervised learning models being relatively difficult to interpret, making it difficult to understand how a decision is made and to infer causes when there are mistakes.

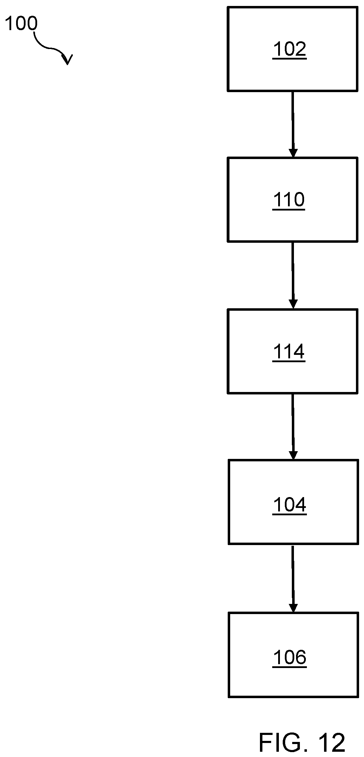

With reference to FIG. 12, the method 100 may comprise receiving 114 an outcome value indicative of an outcome of previous management of the reserve of infant milk. In such embodiments, the determining 104 the infant milk management recommendation may be at least partly based on the outcome value.

Thus, the outcome of the previous management, for example management arising from a previous infant milk management recommendation, may be used as a (partial) basis upon which to determine 104 a contemporaneous infant milk management recommendation. In this way, a reinforcement learning approach may be employed in the determining of the infant milk management recommendation.

In some embodiments, the outcome comprises one or more of: an expiry date of infant milk having elapsed without the infant milk having been fed to an infant; and a measure of quality of a quantity of milk previously recommended for feeding to an infant.

The measure of quality may, for example, be the quality score described above.

In some embodiments, the determining 104 comprises using a reinforcement learning algorithm, such as a model-free reinforcement learning algorithm.

The reinforcement learning algorithm may, for example, operate to increase a number of points, whereby points are earned according to how high the measure of quality is above a threshold, and points are deducted if the measure of quality decreases below the threshold and/or if the expiry date elapses without the infant milk having been fed to the infant.

The model-free reinforcement learning algorithm may, for example, use so-called Q-learning.

Such a Q-learning algorithm may seek to find the best action to take by calculating the maximum expected future rewards for action at each state. In other words, the Q-learning algorithm may find the most optimal balance between a set of given (complex) rules.

The processing system 302 may be configured to pull the required input data from the database 316, and to use the pre-trained reinforcement learning model to determine the infant milk management recommendation.

The quality score of a given quantity of milk may reflect the quality of milk and may therefore have the highest score immediately following expression, and subsequently decrease logarithmically over time as the nutrient amount in the milk decreases, as shown in FIG. 10.

Whereas "first in first out" may only take into account the prevention of milk spoilage and "last in first out" may focus on the delivery of the highest milk quality, the simplest form of a Q-learning algorithm may aim at both achieving the highest milk quality and preventing milk spoilage by considering a holistic strategy including multiple "confounding" parameters/variables.

The decrease in milk quality may be based on different parameters. For example, if the user is following the so-called 666-rule and tracking the storage location of the milk:
- Keeping the milk at room temperature directly after expression results in a score of 0 points after 6 hours.
- Keeping the milk at refrigerator temperature directly after expression results in a score of 0 points after 6 days.
- Keeping the milk at freezer temperature directly after expression results in a score of 0 points after 6 months.

The following Example Scenarios 1 and 2 are provided for illustration:

| Example Scenario 1 | |
|---|---|
| Storage location | Number of quantities |
| Room temp. | 0 |
| Refrigerator temp. | 2 |
| Freezer temp. | 6 |
| Expected expressed milk (next 24 hours) | 2 |
| Expected consumptions (next 24 hours) | 5 |

In Example 1, the infant milk management recommendation may comprise a recommendation to relocate one milk quantity from the freezer to the refrigerator in order to meet demand.

| Example Scenario 2 | |
|---|---|
| Storage location | Number of quantities |
| Room temp. | 1 |
| Refrigerator temp. | 2 |
| Freezer temp. | 2 |
| Expected expressed milk (next 24 hours) | 3 |
| Expected consumptions (next 24 hours) | 3 |

In Example 2, the infant milk management recommendation may comprise a recommendation to relocate two milk quantities from the refrigerator to the freezer and relocate one milk quantity from room temperature to the refrigerator in order to maintain milk quality.

More generally, the method 100 may comprise receiving historic milk expression/consumption data, with the determining 104 being partly based on the historic milk expression/consumption data

For example, the determining, e.g. using the reinforcement learning algorithm, may comprise predicting a future feeding demand, and generate the infant milk management recommendation, e.g. recommending a storage location, based on the predicted future feeding demand.

The infant milk management recommendation may include one or more of the following: (i) recommendations on storage locations, such as relocating one or more milk quantities from one storage location to another storage location to optimise milk quality, and/or relocating milk from one storage location to another storage location for food preparations in the near future, for example relocation from the freezer to the refrigerator for defrosting purposes; and (ii) recommendations on which milk quantity or quantities to pick from the reserve.

It is reiterated that the infant milk management recommendation can be communicated via one or more user interfaces 304, e.g. via a display on a smartphone running a mobile application configured to communicate the infant milk management recommendation; and/or the infant milk management recommendation can be directly visualized via the indicator 202 included in the infant milk container attachment 200.

The various inputs of the system 300 described above in respect of FIGs. 3 and 4 may be collectively termed a "milk tracking unit". Such a milk tracking unit may be configured to monitor, over time, the data indicative of the temperature of each quantity of milk in the reserve, as previously described.

The milk tracking unit may, in some embodiments, track milk entering the reserve (expression moment), and milk leaving the system (consumption moment).

The milk tracking unit may track milk expression data, e.g. moment of expression and milk volume; milk storage data, e.g. storage location, -time, -volume, -temperature (changes); and milk consumption data, e.g. time- and volume of consumption.

This kind of information can be inserted manually by the user via the user interface 304 (e.g. via a mobile application) and/or may be provided by a connected devices, such as breast pump, e.g. a wearable breast pump, and/or the infant milk container attachment 200, that automatically transmit data to the database 316.

The processing system 302 may perform the determining 104 of the infant milk management recommendation, e.g. by the processing system 302 running a reinforcement/self-learning algorithm/model.

The present disclosure may assist in one or more of the following: (i) alleviating the burden on parent(s) and/or caregiver(s) to manually labeling infant milk containers with the expression date, since such timestamp data can be inputted via the user input interface 308 and/or automatically via a connected breast pump and, in some embodiments, displayed via the indicator 202 of the infant milk container attachment 200; (ii) alleviating the burden on parent(s) and/or caregiver(s) in evaluating the milk quality based on different parameters (e.g. storage locations, temperature) and making sure the milk is used before it reaches its expiration date; (iii) alleviating the burden on parent(s) and/or caregiver(s) to relabel the storage bottles/cups/bags manually if milk is placed in different storage locations; and (iv) alleviating the burden on parent(s) and/or caregiver(s) in terms of planning ahead in order to prepare milk on time for future consumption, for example by relocating milk from the freezer to the refrigerator for defrosting.

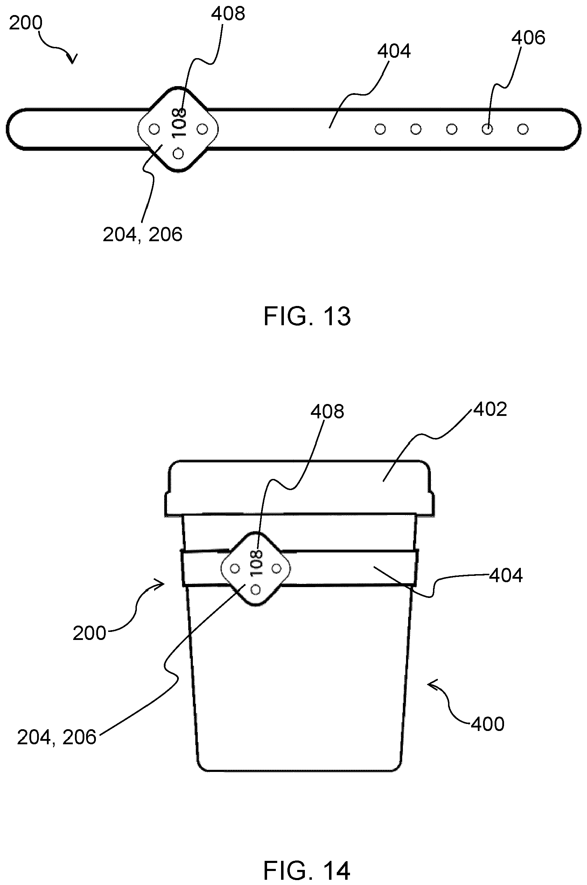

As previously mentioned and referring now to FIGs. 13 and 14, the infant milk container attachment 200 is attachable, e.g. releasably attachable, to an infant milk container 400 in any suitable manner.

An infant milk bottle 400 having a lid 402 is shown in FIG. 14. In this non-limiting example, the infant milk container attachment 200 is attachable via a strap 404 arranged to fasten around the infant milk bottle 400.

Such a strap 404 may delimit one or more holes 406 through which a buckle prong (not visible) is receivable in order to fasten the infant milk container attachment around the infant milk bottle 400.

By the strap 404 delimiting a plurality of holes 406 arranged along the length of the strap 404, the strap 404 may permit attachment of the infant milk container attachment to infant milk bottles 400 having different shapes and/or diameters.

In the non-limiting example shown in FIGs. 13 and 14, the infant milk container attachment 200 is integrated with the strap 404. In other examples, the infant milk container attachment 200 may be provided, e.g. supplied to the user, separately from the strap 404.

In some embodiments, such as that shown in FIGs. 13 and 14, the infant milk container attachment 200 may include an identifier 408, in this case an identifier 408 comprising a numerical identifier, for distinguishing the infant milk container attachment 200, and thus the infant milk bottle 400 to which it is attached, from other milk container attachment(s) 200/infant milk bottle(s) 400 in the reserve of infant milk.

More generally, and as shown in FIGs. 13 and 14, the infant milk container attachment 200 may include the temperature sensor 206 and the communication system 204 configured to communicate temperature sensor data indicative of a temperature of the milk contained in the infant milk bottle 400 to a processing system 302 so as to enable the temperature sensor data to be monitored over time.

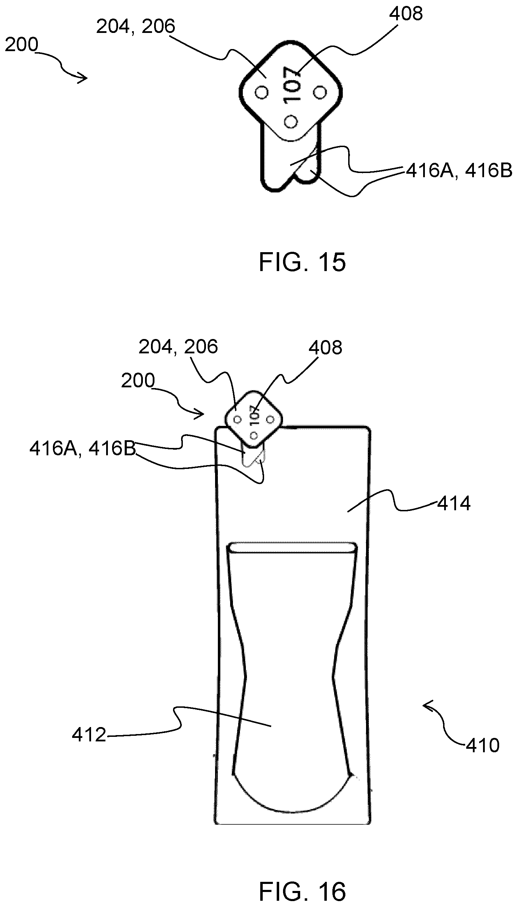

FIGs. 15 and 16 show an infant milk container attachment 200 having a similar design as that shown in FIGs. 13 and 14 other than being attachable to an infant milk storage bag 410.

Such an infant milk storage bag 410 may comprise a pouch 412 defined in a bag body 414. In such embodiments, the infant milk container attachment 200 may be attachable to the bag body 414 via a clip 416A, 416B. The clip 416A, 416B may, for example include a first tab 416A and a second tab 416B, with the attachment being implemented via a portion of the bag body 414 being squeezed between the first tab 416A and the second tab 416B, as shown in FIG. 16.

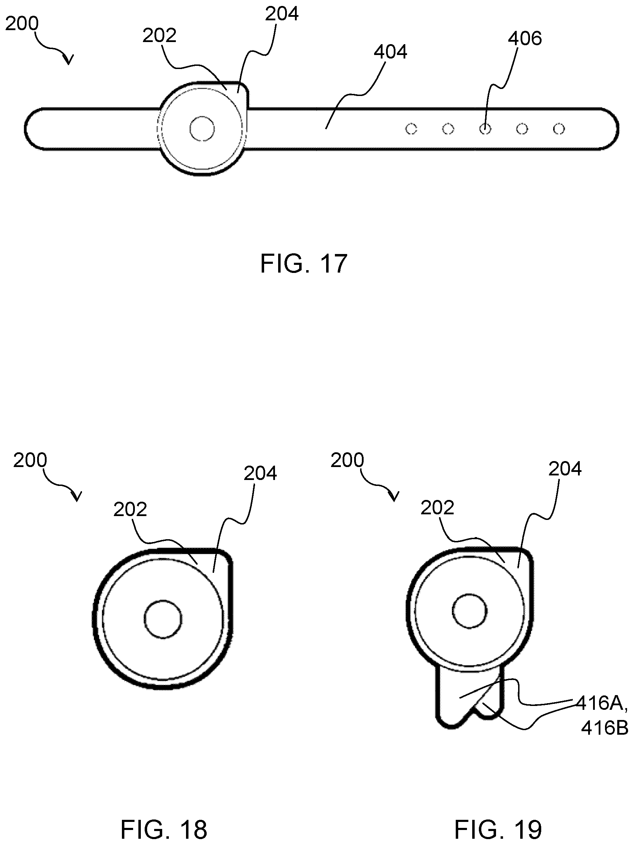

FIGs. 17 to 19 show infant milk container attachments 200 each comprising the indicator 202, in particular an indicator 202 comprising a visual indicator 202, controllable to communicate milk management information. The infant milk container attachments 200 shown in FIGs. 17 to 19 each also include a communication system 204 configured to receive a control signal from a processing system 302, with the indicator 202 being controllable according to the above-described control signal.

The infant milk container attachments 200 shown in FIGs. 17 to 19 may, in some embodiments, also include the temperature sensor 206, with the communication system 204 being further configured to communicate temperature sensor data indicative of a temperature of the milk contained in the infant milk bottle 400 to the processing system 302 so as to enable the temperature sensor data to be monitored over time.

The infant milk container attachment 200 shown in FIG. 17 is attachable via the strap 404 described above in respect of FIGs. 13 and 14, and the infant milk container attachment 200 shown in FIG. 19 is attachable via the clip 416A, 416B described above in respect of FIGs. 15 and 16. The infant milk container attachment 200 shown in FIG. 18 may be attachable via an adhesive layer and/or via magnetic attachment between the infant milk container attachment 200 and the infant milk bottle 400.

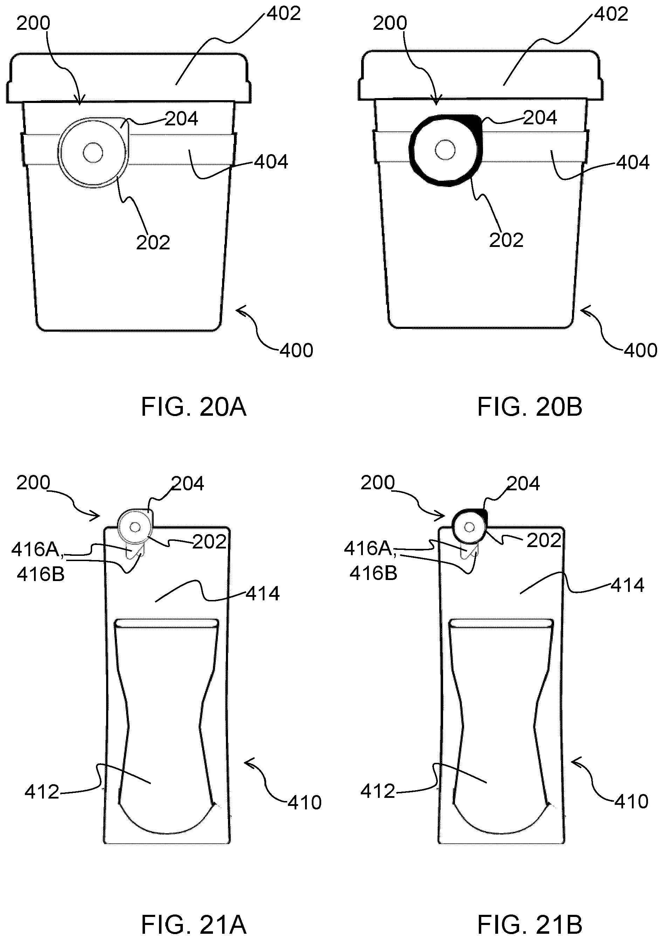

FIGs. 20A and 20B illustrate the indicator 202 being controllable according to the control signal. In FIG. 20A, the indicator 202 is not indicating that the infant milk bottle 400 to which the infant milk container attachment 200 is attached contains a next-to-be-used quantity of milk, in this particular example by the indicator 202 being in the form of a light emitting diode which is not illuminated. However, in FIG. 20B the light emitting diode is controlled via the control signal to illuminate, as represented by the portion denoted by 202 turning from white in FIG. 20A to black in FIG. 20B. This may visually indicate to the user that the infant milk bottle 400 to which the infant milk container attachment 200 is attached contains a next-to-be-used quantity of milk.

FIGs. 21A and 21B provide a similar illustration as FIGs. 20A and 20B, but with the infant milk container attachment 200 being attached to the milk storage bag 410.

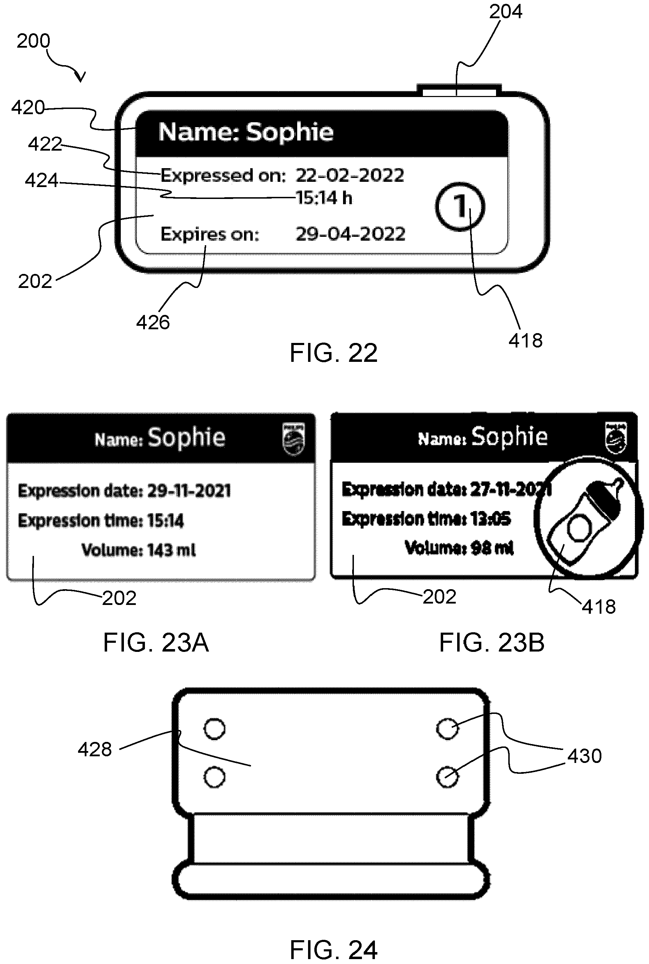

In the non-limiting example shown in FIG. 22, the indicator 202 comprises an electronic-ink display for displaying the infant milk management recommendation 418. In this example, the infant milk management recommendation 418 comprises an indication of the order in which the quantity of milk in the infant milk bottle 400 to which the infant milk container attachment 200 is attached is to be fed to an infant. In this case, the "1" indicates that the quantity of milk in the infant milk bottle 400 to which the infant milk container attachment 200 is the next-to-be fed quantity.

In some embodiments, such as that shown in FIG. 22, the indicator 202 is controllable, e.g. by the processing system 202, to display one or more of: a name 420 of the infant to whom the quantity of milk is to be fed, a date 422 of expression of the milk, a time 424 of expression of the milk, an amount, e.g. a mass and/or volume, of the quantity of milk, a quality, e.g. a calculated quality score, of the milk, an indication 426 of when/whether or not the milk has expired, a location of the infant milk container, and the indication 418 of the order in which the quantity of milk is to be fed to the infant.

In embodiments, in which the infant milk container attachment 200 comprises the temperature sensor 206, the communication system 204 may be configured to periodically communicate the temperature sensor data, e.g. by periodically uploading the temperature sensor data to the database 316, so as to enable the processing system 302 to monitor 102 the temperature sensor data overtime.

The infant milk container attachment 200 can be configured in various ways in order to facilitate monitoring of the temperature sensor data over time.

The temperature sensor 206 may be configured to collect the temperature sensor data at a sample rate, and communicate the collected temperature sensor data periodically to the database 316 and thus to the processing system 302.

In some embodiments, the collected temperature sensor data is communicated at a lower frequency than the sample rate.

This may assist to reduce power consumption, e.g. so as to preserve the operating lifetime of a battery powering the infant milk container attachment 200.

For example, the sample rate may be every minute, and the collected temperature sensor data may be communicated 10 times per day. In such an example, communicating the temperature sensor data wirelessly, e.g. via Bluetooth^{®}, can mean that the operating lifetime of the battery is around 10 months.

Alternatively or additionally, the sampling and/or the communicating may be based on the sensed temperature conditions.

For example, a variable sample rate may vary in accordance with the sensed temperature conditions.

For instance, sampling may be triggered by a change in the temperature.

In some embodiments, the temperature sensor data may only be communicated in response to a specific event, for example when the infant milk container, to which the infant milk container attachment 200 is attached, is used.

FIGs. 23A and 23B illustrate the indicator 202 being controllable according to the control signal. In FIG. 23A, the indicator 202 is not indicating that the infant milk container to which the infant milk container attachment 200 is attached contains a next-to-be-used quantity of milk. However, in FIG. 23B the indicator 202 is controlled via the control signal such that a symbol 418 is displayed by the electronic-ink display in this example. The appearance of this symbol 418 may visually indicate to the user that the infant milk container to which the infant milk container attachment 200 is attached contains a next-to-be-used quantity of milk.

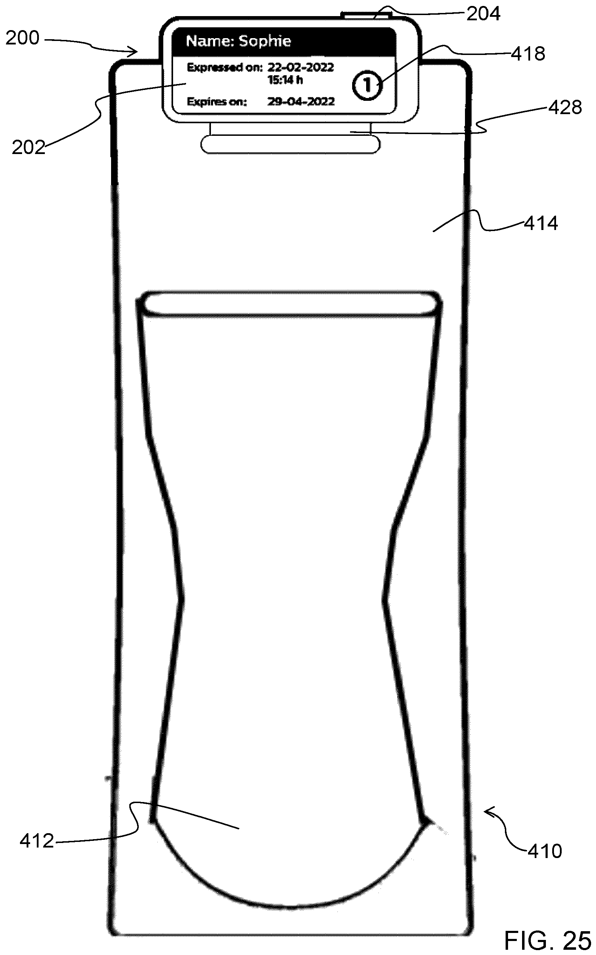

FIG. 24 shows an attachment member 428 for attaching the infant milk container attachment 200 shown in FIGs. 22 to 23B to an infant milk container, in particular to an infant milk container in the form of a milk storage bag 410. Referring also to FIG. 25, a portion of the bag body 414 may be sandwiched between a container-facing surface of the infant milk container attachment 200 and the attachment member 428.

The attachment member 428 may, for example, comprise one or more magnetic portions 430 arranged to attract the container-facing surface of the infant milk container attachment 200 and thereby trap the portion of the bag body 414 between the container-facing surface and the attachment member 428.

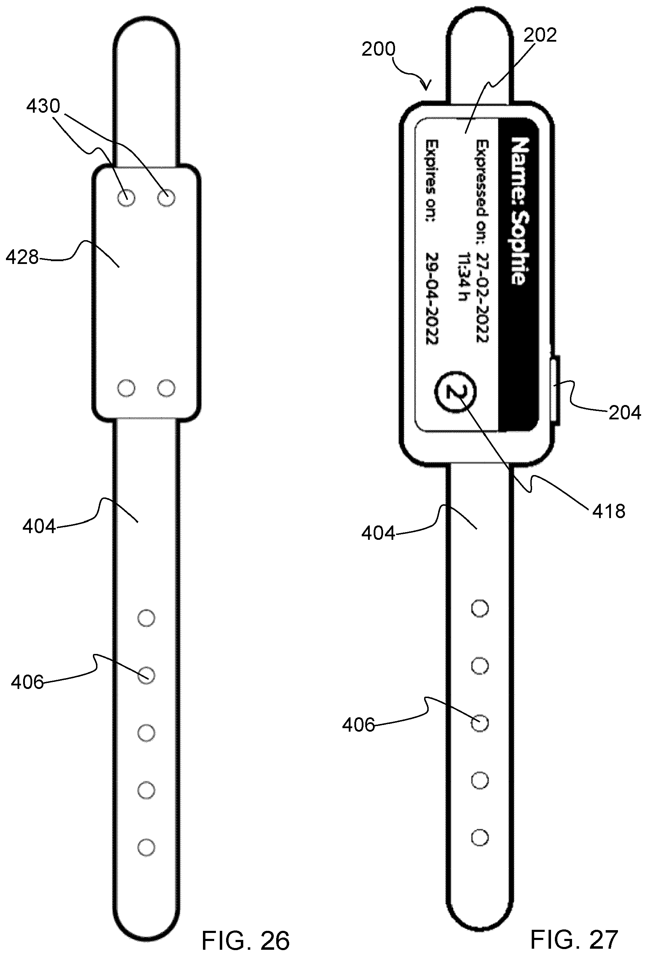

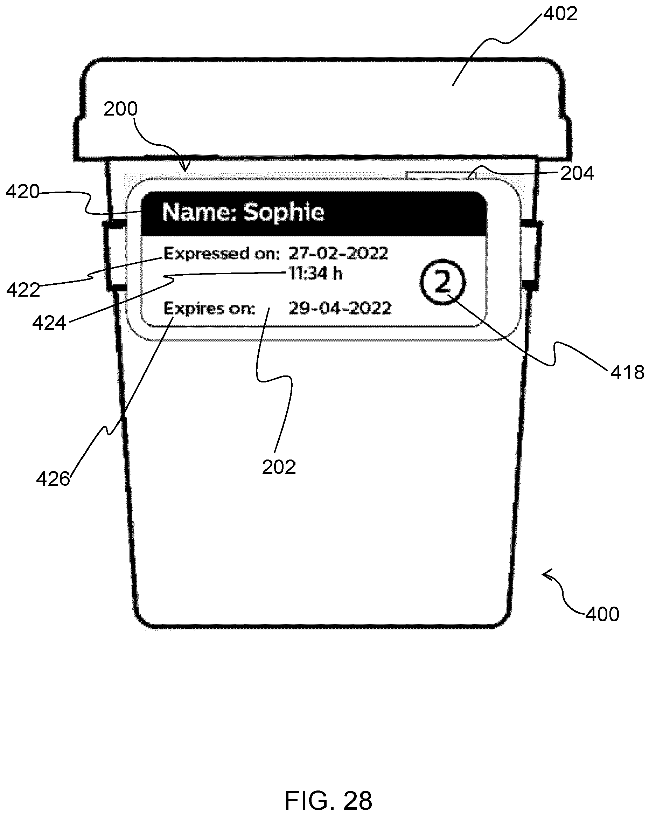

In the non-limiting example shown in FIGs. 26 to 28, the attachment member 428 is attachable via the strap 404 around the infant milk bottle 400, and the infant milk container attachment 200 is itself attachable to the attachment member 428, e.g. magnetically as described above in respect of the example shown in FIGs. 24 and 25.

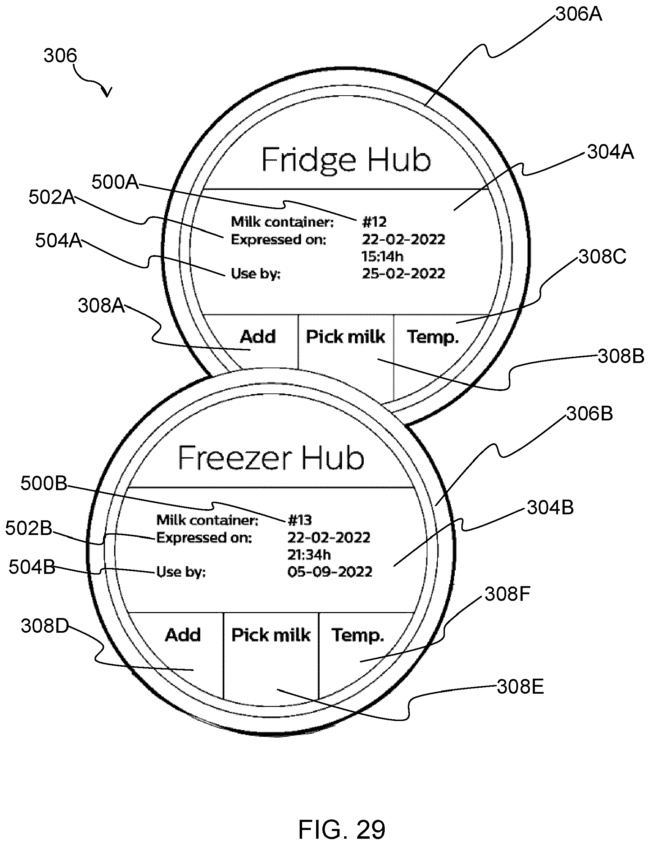

In some embodiments, such as that shown in FIG. 29, the user device 306 includes a refrigerator display 304A mountable on a refrigerator door and/or a freezer display 304B mountable on a freezer door.

The refrigerator display 304A and/or the freezer display 304B may be controllable to indicate the next-to-be-used quantity or quantities in the refrigerator and/or the freezer to distinguish them from a remainder of the reserve of infant milk. The indicator of the next-to-be used quantities is denoted in FIG. 29 by reference numerals 500A and 500B.

In the non-limiting example shown in FIG. 29, timestamp data 502A, 502B indicating when each of the next-to-be-used quantities of milk was expressed, and a use by date 504A, 504B for each of the next-to-be-used quantities of milk is also shown via the refrigerator display 304A and the freezer display 304B.

The user device 306, e.g. "refrigerator hub" 306A, including the refrigerator display 304A may further include a user input interface 308A, 308B, 308C.

The user device 306, e.g. "freezer hub" 306B, including the freezer display 304B may further include a user input interface 308D, 308E, 308F.

The user input interface 308A may be actuated by a user when adding one or more further quantities of milk to the reserve, and in particular to a refrigerator reserve stored in the refrigerator.

Similarly, the user input interface 308D may be actuated by a user when adding one or more further quantities of milk to the reserve, and in particular to a freezer reserve stored in the refrigerator.

The user input interface 308B may be actuated by a user when picking one or more quantities of milk from the reserve, and in particular from the refrigerator reserve stored in the refrigerator, for feeding to an infant.

Similarly, the user input interface 308E may be actuated by a user when picking one or more quantities of milk from the reserve, and in particular from the freezer reserve stored in the freezer, for defrosting and then feeding to an infant.

The user input interface 308C may be actuated by a user wishing to check a temperature inside the refrigerator, e.g. temperature sensor data from one or more infant milk container attachments 200 located inside the refrigerator.

Similarly, the user input interface 308F may be actuated by a user wishing to check a temperature inside the freezer, e.g. temperature sensor data from one or more infant milk container attachments 200 located inside the freezer.

In embodiments in which the method 100 further comprises receiving an amount of milk in each said quantity of milk of the reserve of infant milk, with the determining 104 the infant milk management recommendation being partly based on said amount of milk, the amount of milk may be received from a scale 600 on which an infant milk container containing the respective quantity of milk is placed to measure the amount of milk. Such a scale 600 is shown in FIG. 30.

The scale 600 may include a weighing plate 601 on which the infant milk container is receivable in order to weigh the quantity of milk contained therein.

In such embodiments, the method 100 may include converting, using a density of the milk, a mass of the milk into a volume of the milk.

In some embodiments, the method 100 further comprises receiving an amount of milk to be fed to an infant.

Such an amount of milk to be fed may, for example, be an amount of milk to be fed to the infant in a next feed, or an amount of milk to be fed to an infant over a given time period, for example while the infant is attending their childcare setting.

In such embodiments, the determining 104 the infant milk management recommendation may comprise determining next-to-be-used quantity or quantities of milk of the reserve of infant milk based on the amount of milk in each said quantity and the amount of milk to be fed to the infant.

In such embodiments, the indicated next-to-be-used quantity or quantities of milk of the reserve of infant milk may correspond to an amount of milk which equals or exceeds the amount of milk to be fed to the infant.

More generally, the infant milk management recommendation may comprise a recommendation to use a combination of quantities in order to get the desired amount of milk. In a practical example, if the infant needs to go to daycare the following day, the user may be required to prepare the right amount of milk to last for the full day. The determining 104 may comprise estimating which is the best combination of milk quantities to provide the right amount of milk with the best quality and least waste, and the next-to-be-used quantities may be indicated accordingly.

In some embodiments, the indicator 202 of each of the next-to-be-used quantities may indicate that it is part of a group of next-to-be-used quantities. For example, the indicator 202 may show 1/3, 2/3, 3/3 in the case that there are three next-to-be-used quantities.

Returning to the non-limiting example shown in FIG. 30, the scale 600 comprises a user interface 602, 604 comprising a user input interface configured to enable the scale 600 to be switched on and/or tared, e.g. when an empty infant milk container is placed on the weighing plate 601.

Alternatively or additionally, the user interface 602, 604 may include a user output interface configured to communicate the amount, e.g. volume and/or mass, of the quantity of milk in the infant milk container to the user, along with an identifier for identifying the quantity.

Such an identifier may, for example, be assigned to the quantity contained in one of the infant milk containers via the numbered labels 606 shown in FIG. 31, with each label 606 having a different number 608 from the others.

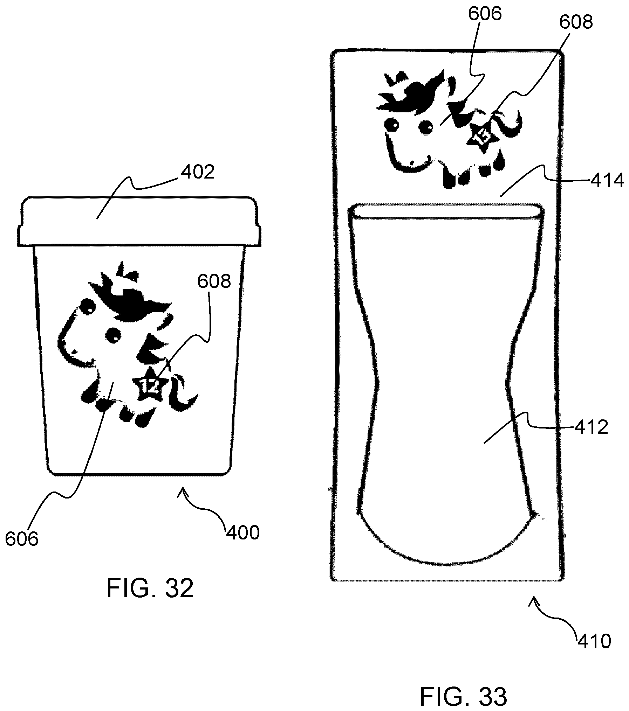

Such labels 606 may be attachable, e.g. adhesively attachable, to an infant milk bottle 400, as shown in FIG. 32, and/or to a milk storage bag 410, as shown in FIG. 33.

The labels 606 may, for example, include one or both of the above-described temperature sensor 206 and indicator 202.

In embodiments in which the scale 600 is connectable to the processing system 302, the connected scale may make it easier for the user to portion their milk and to keep track of volumes.

The labels 606, which can be regarded as unique stickers, can be recognized by the scale 600 while the milk is being weighed.

This may assist the user in assigning the quantity being weight to a storage location.

The user output interface 308 of the user device 306 may, for example, communicate the next-to-be-used quantity or quantities by displaying the unique number 608, e.g. code, of the respective label 606 attached to each of the infant milk containers containing the next-to-be-used quantity or quantities.

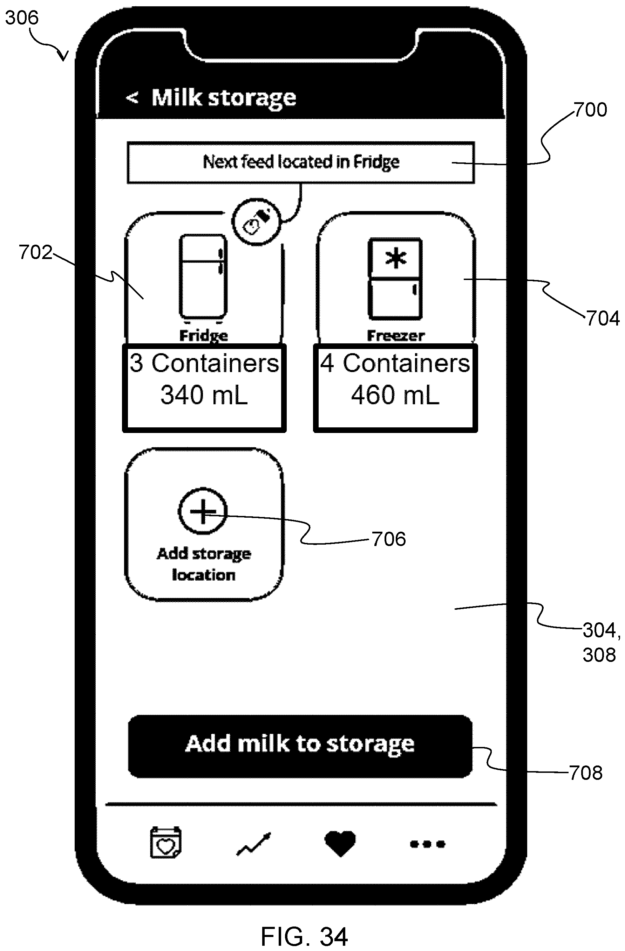

FIGs. 34 to 36 provide screenshots of a smartphone according to an example, an in particular screenshots of the above-mentioned application loaded onto the smartphone.

The smartphone may be an example of the above-mentioned user device 306.

In the screenshot shown in FIG. 34, the infant milk management recommendation comprises an indication 700 of the storage location of the next-to-be-used quantity of milk, in this case an indication 700 that the next-to-be-used quantity of milk is located in the refrigerator.

In the non-limiting example shown in FIG. 34, the screenshot further comprises an indication 702 of the number of infant milk containers, and the total volume of milk contained in the infant milk containers, stored in the refrigerator. Similarly, the screenshot comprises an indication 704 of the number of infant milk containers, and the total volume of milk contained in the infant milk containers, stored in the freezer.

The smartphone's touchscreen may further provide user input functionality, and in the screenshot shown in FIG. 34, a button 706 is included permitting the user to add a storage location to the existing storage locations, in this example the refrigerator and the freezer, in which to store at least some of the reserve of infant milk.

A further button 708 may also be provided which enables the user to register a further milk quantity into the reserve of infant milk.

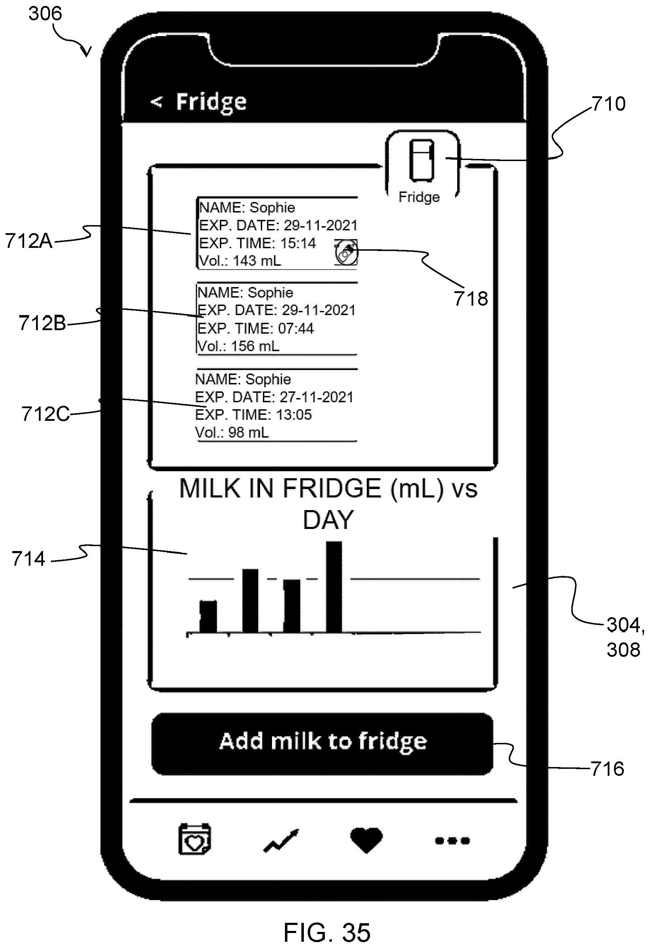

The screenshot shown in FIG. 35 provides details concerning the refrigerator-stored portion 710 of the infant milk reserve. Details for each of the refrigerator stored quantities 712A, 712B, 712C are provided, together with a graph 714 showing a trend of milk in the refrigerator vs time in days. The screenshot shown in FIG. 35 also includes a button 716 which enables the user to register a further milk quantity into the refrigerator-stored portion of the reserve of infant milk.

Further evident in FIG. 35 is the indication 718 of an order in which quantities of the milk are to be fed to an infant. The symbol 718 denotes the next-to-be-used quantity.

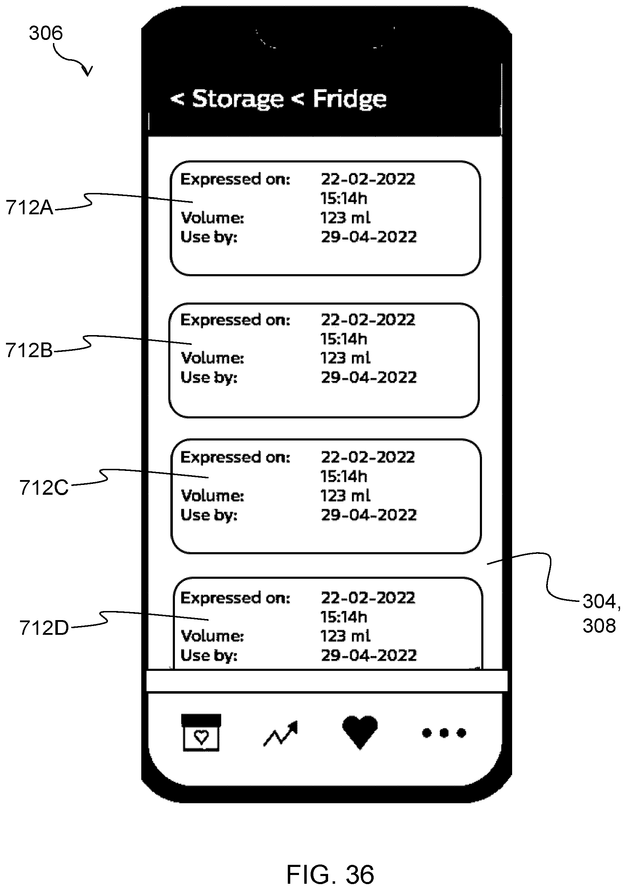

FIG. 36 shows another example of an indication of an order in which quantities of the milk are to be fed to an infant. In this case, the details for each of the refrigerator stored quantities 712A, 712B, 712C, 712D are shown from a top to a bottom of the screen in the order in which the quantities should be used. Such details may also correspond with those communicated via an indicator 202, e.g. an electronic-ink display, included in an infant milk container attachment 200 attached to the infant milk container containing the respective quantity of milk.

When the infant milk management recommendation is made, the processing system 302 may control the indicator 202 of the infant milk container attachment 200 to display a sign that the quantity contained in the infant milk container to which that infant milk container 200 is attached is the next-be-used, as previously described. Such a recommendation can also be provided via the user output interface 304 included in the user device 306, e.g. via an application run on a smartphone.

The information provided via the application may contain, for example, the storage location of the infant milk container(s) containing the next-to-be-used quantity or quantities.

A copy of the image also displayed via the indicator 202 of the infant milk container attachment 200 attached to the container may also be presented via the application. Thus, any member of the family may be able to select the recommended quantity/infant milk container at any point in time. The recommended quantity/container may be found directly by looking in the refrigerator/freezer or by first checking on the application for where to find it.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by the processing system 302 may be implemented by a single processor or by multiple separate processing units which may together be considered to constitute a "processing system". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A computer implemented method (100) for communicating an infant milk management recommendation to a user, the method comprising:
monitoring (102), over time, data indicative of a temperature of each quantity of milk in a reserve of infant milk;
determining (104) said infant milk management recommendation at least partly based on said data monitored over time; and
communicating (106) the infant milk management recommendation to the user.

2. The method according to claim 1, wherein said data comprises temperature sensor data generated by at least one temperature sensor; optionally wherein a temperature sensor is associated with each said quantity of milk so as to generate temperature sensor data indicative of a temperature of the respective quantity of milk associated with the temperature sensor.

3. The method (100) according to claim 1 or claim 2, further comprising receiving (110) timestamp data indicating when each said quantity of milk was expressed, the determining (104) said infant milk management recommendation being partly based on said timestamp data; optionally wherein said timestamp data comprises a date and/or a time of expression.

4. The method (100) according to any of claims 1 to 3, further comprising receiving (114) an outcome value indicative of an outcome of previous management of the reserve of infant milk, the determining (104) said infant milk management recommendation being partly based on said outcome value.

5. The method (100) according to claim 4, wherein said outcome comprises one or more of:
an expiry date of infant milk having elapsed without the infant milk having been fed to an infant; and
a measure of quality of a quantity of milk previously recommended for feeding to an infant.

6. The method (100) according to claim 4 or claim 5, wherein the determining (104) said infant milk management recommendation comprises using a reinforcement learning algorithm; optionally a model-free reinforcement learning algorithm.

7. The method (100) according to any of claims 1 to 6, further comprising receiving (112) a user preference value indicative of the user's preference for managing the reserve of infant milk, the determining said infant milk management recommendation being partly based on said user preference value; optionally wherein said user preference value is indicative of the user's preference for either minimising the risk of milk spoilage or maximising freshness of milk taken from the reserve of infant milk to feed to an infant.

8. The method according to any of claims 1 to 7, wherein said infant milk management recommendation comprises an indication of an order in which quantities of the milk are to be fed to an infant.

9. The method according to claim 8, wherein said communicating comprises controlling one or more user interfaces to indicate next-to-be-used quantity or quantities of milk of the reserve of infant milk.

10. The method according to claim 9, wherein said one or more user interfaces comprises an indicator associated with each said quantity of milk, said communicating comprising selectively controlling the indicator or indicators of the next-to-be-used quantity or quantities to distinguish them from a remainder of the reserve of infant milk.

11. The method according to any of claims 1 to 10, further comprising receiving an amount of milk in each said quantity of milk of the reserve of infant milk, the determining said infant milk management recommendation being partly based on said amount of milk; optionally wherein said amount is a volume and/or a mass of each said quantity of milk.

12. The method according to any of claims 1 to 11, wherein said infant milk management recommendation comprises a recommendation of a storage temperature condition for storing one or more further milk quantities to be added to the reserve of infant milk; and/or wherein said infant milk management recommendation comprises a recommendation of a change to a storage temperature condition of at least one milk quantity in the reserve of infant milk.

13. A computer program comprising computer program code which, when executed on a computing device having a processing system, causes the processing system to perform all of the steps of the method (100) according to any of claims 1 to 12.

14. An infant milk container attachment (200) comprising:
a temperature sensor (206) arrangeable on and/or in an infant milk container; and
a communication system (204) adapted to communicate temperature sensor data from the temperature sensor to a processing system so as to enable the temperature sensor data to be monitored over time.

15. An infant milk management system (300) comprising:
a processing system (302) configured to:
monitor, over time, data indicative of a temperature of each quantity of milk in a reserve of infant milk;
determine said infant milk management recommendation at least partly based on said data monitored over time; and
control one or more user interfaces to communicate the infant milk management recommendation to the user; optionally wherein said data comprises temperature sensor data generated by at least one temperature sensor (206A, 206B, 206C, 206D).
